(19) Europäisches Patentamt
European Patent Office
Office européen des brevets

(11) Veröffentlichungsnummer: **0 074 018**
**B1**

# (12) EUROPÄISCHE PATENTSCHRIFT

(45) Veröffentlichungstag der Patentschrift: **24.09.86**

(21) Anmeldenummer: **82107789.8**

(22) Anmeldetag: **25.08.82**

(51) Int. Cl.[4]: **C 07 D 213/30**, C 07 D 241/12, C 07 D 213/26, C 07 D 213/32, C 07 D 213/89, C 07 D 213/50, C 07 D 213/57, C 07 D 405/04, A 01 N 43/40, A 01 N 43/60

(54) **Pyridin- und Pyrazin-Derivate, Herstellung dieser Verbindungen, fungizide Mittel, die diese Verbindungen als Wirkstoffe enthalten, Verwendung solcher Verbindungen bzw. Mittel zur Bekämpfung von Fungi in der Landwirtschaft und im Gartenbau sowie Ketone und diese enthaltende fungizide Mittel.**

(30) Priorität: **01.09.81 CH 5622/81**
**09.07.82 CH 4206/82**

(43) Veröffentlichungstag der Anmeldung: **16.03.83 Patentblatt 83/11**

(45) Bekanntmachung des Hinweises auf die Patenterteilung: **24.09.86 Patentblatt 86/39**

(84) Benannte Vertragsstaaten: **AT BE CH DE FR GB IT LI NL**

(56) Entgegenhaltungen:
**Die Akte enthält technische Angaben, die nach dem Eingang der Anmeldung eingereicht wurden und die nicht in dieser Patentschrift enthalten sind.**

(73) Patentinhaber: **F. HOFFMANN- LA ROCHE & CO. Aktiengesellschaft, CH- 4002 Basel (CH)**

(72) Erfinder: **Dorn, Franz, Dr., Bohnackerstrasse 5, CH- 8157 Dielsdorf (CH)**
Erfinder: **Montavon, François, Dr., rue des Romains 4, CH- 2800 Delémont (CH)**
Erfinder: **Suchy, Milos, Dr., Grossplatzstrasse 3, CH- 8122 Pfaffhausen (CH)**

(74) Vertreter: **Lederer, Franz, Dr., Vanderwerth, Lederer & Riederer Patentanwälte Lucile- Grahn- Strasse 22, D-8000 München 80 (DE)**

**EP 0 074 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft heterocyclische Verbindungen, insbesondere Pyridin- und Pyrazin-Derivate der allgemeinen Formel I

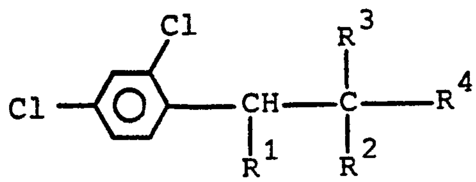

I

worin $R^1$ Halogen, $C_{1-4}$-Alkyl, $OR^5$, $SR^5$ oder $OSO_2R^5$,
$R^2$ Hydroxy oder Chlor,
$R^3$ Wasserstoff oder $C_{1-4}$-Alkyl,
$R^4$ 3-Pyridyl, 3-Pyridyl-1-oxid, 2-Pyrazinyl, 2-Pyrazinyl-1-oxid, 2-Pyrazinyl-4-oxid oder 2-Pyrazinyl-1,4-dioxid
und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann, und deren Säureadditionssalze.

Die Verbindungen der Formel I und deren Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner Verfahren zur Herstellung der Verbindungen der Formel I und ihrer Säureadditionssalze, fungizide Mittel, die Verbindungen der Formel I oder Säureadditionssalze davon als Wirkstoffe enthalten, sowie die Verwendung solcher Verbindungen, Säureadditionssalze und Mittel zur Bekämpfung von Pilzen in der Landwirtschaft und im Gartenbau.

Die Schweizerische Patentschrift Nr. 498.568 beschreibt u.a. fungizid wirksame Verbindungen der Formel

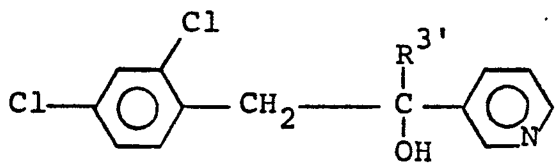

sowie deren N-Oxide und Säuradditionssalze. Alle hier angesprochenen Verbindungen weisen ausnahmslos eine unsubstituierte Methylenbrücke und als Rest $R^3$ entweder Aryl oder Aralkyl auf.

Die DOS 1.913.726 beschreibt u.a. ein einziges entsprechendes, ebenfalls fungizid wirksames Pyrazinderivat, Wobei der Rest $R^{3'}$ Phenyl ist und auch der Benzylrest unsubstituiert ist.

Der Ausdrück "Halogen" in der Definition der Formel I umfasst Fluor, Chlor, Brom und Jod.

Die Ausdrücke "$C_{1-4}$-Alkyl" umfassen sowohl geradkettige als auch verzweigte Alkylgruppen, so dass folgende Gruppen zu verstehen sind: Methyl, Aethyl, n-Propyl, Isopropyl, n-Butyl, sek. Butyl, Isobutyl und tert. Butyl.

$R^1$ in der Bedeutung Halogen ist vorzugsweise Chlor oder Brom. Bedeutet $R^1$ $C_{1-4}$-Alkyl, so ist dies vorzugsweise Methyl.

$R^3$ bedeutet vorzugsweise Wasserstoff oder Methyl.

Besonders bevorzugte Verbindungen der Formel I sind:
α-(α,2,4-Trichlorbenzyl)-α-methyl-3-pyridylmethanol,
α-(2,4-Dichlor-α-methylbenzyl)-3-pyridylmethanol,
α-(α-Brom-2,4-dichlorbenzyl)-α-methyl-2-pyrazinylmethanol
und α-(α-Brom-2,4-dichlorbenzyl)-α-methyl-3-pyridylmethanol.

Weitere Vertreter von Verbindungen der Formel I sind:
α-(α,2,4-Trichlorbenzyl)-3-pyridylmethanol,
α-(α-Brom-2,4-dichlorbenzyl)-3-pyridylmethanol,
α-(2,4-Dichlor-α-methoxybenzyl)-3-pyridylmethanol
und α-(2,4-Dichlor-α-methoxybenzyl)-α-methyl-2-pyrazinylmethanol.

Das Vorhandensein von mindestens zwei asymmetrischen Kohlenstoffatomen in den Verbindungen der Formel I hat zur Folge, dass die Verbindungen in isomeren Formen auftreten können, welche in enantiomerer oder diastereomerer Beziehung zueinander stehen. Die Formel I soll demnach die entsprechenden enantiomeren und diastereomeren Formen umfassen.

Als Säureadditionssalze der Verbindungen der Formel I kommen physiologisch verträgliche Salze in Frage. Hierzu gehören vorzugsweise Salze dieser Verbindungen mit anorganischen und organischen Säuren, wie Salzsäure; Salpetersäure; Phosphorsäure; mono- und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure; und Sulfonsäuren, z.B. 1,5-Naphthalin-disulfonsäure.

Das erfindungsgemässe Verfahren zur Herstellung der Verbindungen der Formel I und von deren

Säureadditionssalzen ist dadurch gekennzeichnet, dass man.

a) zwecks Herstellung der Verbindungen der Formel I, worin. $R^1$ Halogen, $OR^5$, $SR^5$ oder $OSO_2R^5$, $R^2$ Hydroxy und $R^4$ 3-Pyridyl oder 2-Pyrazinyl bedeuten, ein Epoxid der allgemeinen Formel

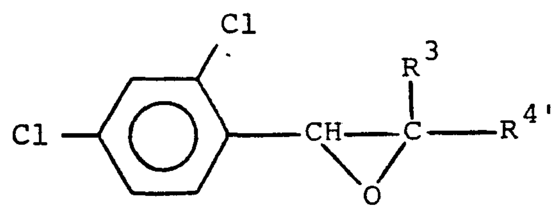

II

worin $R^3$ die oben angegebene Bedeutung besitzt

und $R^{4'}$ 3-Pyridyl oder 2-Pyrazinyl bedeutet oder einen Alkohol der allgemeinen Formel

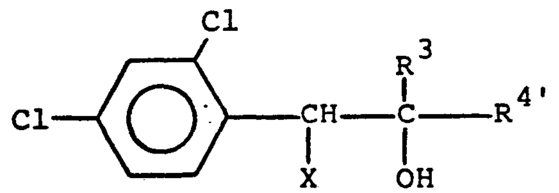

III

worin $R^3$ und $R^4$ die oben angegebenen Bedeutungen besitzen

und X einen Alkylsulfonatrest, z.B. den Methansulfonatrest, oder Arylsulfonatrest, z.B. den Benzolsulfonat- oder p-Toluolsulfonatrest, bedeutet, mit einer Verbindung der allgemeinen Formel

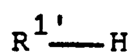

IV

worin $R^{1'}$ Halogen, $OR^5$, $SR^5$ oder $OSO_2R^5$ bedeutet

und $R^5$ die oben angegebene Bedeutung besitzt, umsetzt,

b) zwecks Herstellung der Verbindungen der Formel I, worin $R^1$ $C_{1-4}$-Alkyl, $R^2$ Hydroxy, $R^3$ wasserstoff und $R^4$ 3-Pyridyl oder 2-Pyrazinyl bedeuten, ein Keton der allgemeinen Formel

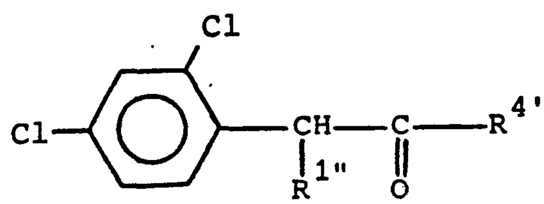

V

worin $R^{4'}$ die oben angegebene Bedeutung besitzt

und $R^{1''}$ $C_{1-4}$-Alkyl bedeutet reduziert

c) zwecks Herstellung der Verbindungen der Formel I, worin $R^1$ $C_{1-4}$-Alkyl, $R^2$ Hydroxy, $R^3$ $C_{1-4}$-Alkyl und $R^4$ 3-Pyridyl oder 2-Pyrazinyl bedeuten, ein Keton der allgemeinen Formel V, wie oben definiert, mit einer Verbindung der allgemeinen Formel

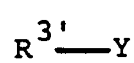

VI

worin $R^{3'}$ $C_{1-4}$-Alkyl,

Y Lithium oder MgZ

und Z Halogen, insbesondere Brom oder Jod, bedeuten,

umsetzt,

d) zwecks Herstellung der Verbindungen der Formel I, worin $R^2$ Chlor und $R^4$ 3-Pyridyl oder 2-Pyrazinyl bedeuten, einen Alkohol der allgemeinen Formel

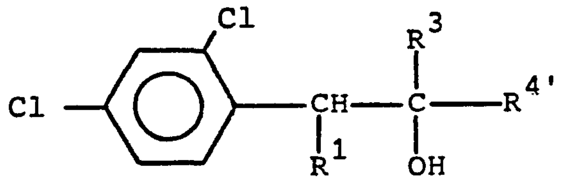

I'

worin $R^1$, $R^3$ und $R^4$ die oben angegebenen Bedeutungen besitzen,

oder ein Epoxid der allgemeinen Formel II wie oben definiert, mit einem Chlorierungsmittel behandelt,

e) zwecks Herstellung der Verbindungen der Formel I, worin $R^1$ $SR^5$ und $R^2$ Hydroxy bedeuten, einen Thioäther der allgemeinen Formel

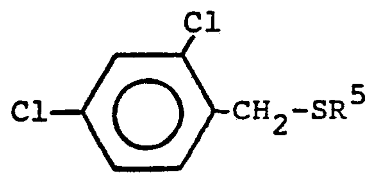

VII

worin $R^5$ die oben angegebene Bedeutung besitzt mit einem Aldehyd bzw. Keton der allgemeinen Formel

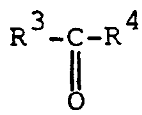

VIII

worin $R^3$ und $R^4$ die oben angegebenen Bedeutungen besitzen,

umsetzt, oder

f) zwecks Herstellung der Verbindungen der Formel I, worin $R^4$ pyridyl-1-oxid, 2-Pyrazinyl-1-oxid, 2-Pyrazinyl-4-oxid oder 2-Pyrazinyl-1,4-dioxid bedeutet, ein Pyridin- bzw. Pyrazin-Derivat der allgemeinen Formel

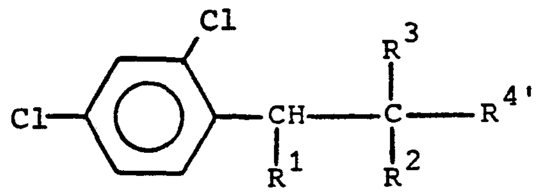

I"

worin $R^1$ $R^2$, $R^3$ und $R^4$ die oben angegebenen Bedeutungen besitzen,

N-oxydiert

und erwünschtenfalls eine erhaltene Verbindung der Formel I mit einer Säure zur Herstellung des entsprechenden Säureadditionssalzes umsetzt.

Bei der Verfahrensvariante a) wird zweckmässigerweise mit einem Ueberschuss der Verbindung der Formel IV und in einem Temperaturbereich zwischen 0°C und der Rückflusstemperatur des Reaktionsgemisches gearbeitet. Im Falle der Umsetzung mit dem Epoxid der Formel II beträgt der bevorzugte Temperaturbereich 0°C bis 70°C und der bevorzugte Ueberschuss der Verbindung der Formel IV bis zu 200 Gewichtsprozent. Man arbeitet im Falle der Umsetzung mit dem Alkohol der Formel III vorzugsweise in einem Temperaturbereich zwischen der Raumtemperatur und der Rückflusstemperatur des Reaktionsgemisches. Im Falle der Umsetzung des Alkohols III mit der Verbindung IV kann die letztere auch als Alkalimetall- oder Erdalkalimetallsalz eingesetzt werden.

Zudem kann in beiden Fällen die Umsetzung in einem Verdünnungsmittel erfolgen. Wird ein Verdünnungsmittel benützt, so ist dies im ersten Fall vorzugsweise das im Ueberschuss verwendete Reagens Hal-H $R^5OH$ bzw. $R^5SH$ oder ein inertes organisches Lösungsmittel, wie ein aromatischer Kohlenwasserstoff, z.B. Benzol oder Toluol; ein Aether oder eine ätherartige Verbindung, z.B. Diäthyläther, Dioxan oder Tetrahydrofuran; ein halogenierter aliphatischer Kohlenwasserstoff, z.B. Methylenchlorid, Chloroform oder Tetrachlorkohlenstoff; oder Dimethylformamid; oder Wasser. Im zweiten Fall ist dies vorzugsweise eines der oben aufgeführten inerten organischen Lösungsmittel oder Wasser.

In denjenigen Fällen, in denen das Reagens der Formel IV selbst keine starke Säure darstellt, d.h. einen $pK_s$-Wert höher als ca. 5 aufweist, ist für die Reaktion mit dem Epoxid der Formel II die Zugabe einer Mineralsäure, z.B. Schwefelsäure, oder einer Sulfonsäure, z.B. p-Toluolsulfonsäure, von Vorteil. Dies gilt insbesondere für den Fall dass das Reagens der Formel IV ein niederer Alkohol oder ein niederes Mercaptan ist, d.h. $R^{1'}$ in der Formel IV $OR^5$ oder $SR^5$ darstellt.

Bei der Umsetzung mit dem Alkohol der Formel III resultiert eine Inversion am die 2,4-Dichlorphenylgruppe und den Rest X tragenden Kohlenstoffatom, so dass z.B. ein Alkohol der Formel III, worin X die Gruppe $OSO_2CH_3$ bedeutet, mit Methansulfonsäure als Verbindung der Formel IV umgesetzt werden kann, um die andere enantiomere Form des Alkohols der Formel III herzustellen.

Beispiele von in dieser Verfahrensvariante gegebenenfalls verwendeten Salzen der Verbindung der Formel IV sind Alkalimetallsalze, wie die Natrium- und Kaliumsalze, und Erdalkalimetallsalze, wie die Calcium- und Magnesiumsalze.

Es erweist sich als vorteilhaft, den Alkohol der Formel III mittels Umsetzung des entsprechenden Epoxids der Formel II mit einer Alkyl- bzw. Arylsulfonsäur, z.B. einer Verbindung der Formel IV, worin $R^1$ $OSO_{2R5}$ bedeutet, in situ herzustellen und anschliessend den resultierenden Alkohol der Formel III ohne Isolierung mit einer Verbindung der Formel IV oder einem Salz davon zum Endprodukt weiterzureagieren.

Die Reduktion gemäss Verfahrensvariante b) erfolgt vorzugsweise mittels eines komplexen Metallhydrids, wie Natriumborhydrid, wobei in einem protischen Verdünnungsmittel, wie einem Alkohol, z.B. Methanol oder Aethanol, bei Temperaturen um Raumtemperatur gearbeitet wird, oder Lithiumaluminiumhydrid, das in einem aprotischen Verdünnungsmittel, insbesondere einem Aether oder einer ätherartigen Verbindung, z.B. Diäthyläther, Tetrahydrofuran oder Dioxan, bei Reaktionstemperaturen zwischen 0°C und der Raumtemperatur eingesetzt wird. Als komplexe Metallhydride in geeigneten Lösungsmitteln kommen auch Lithiumborhydrid in Aethanol oder Tetrahydrofuran, Natriumborhydrid-Aluminiumchlorid in einem Aether, z.B. Diglym, und Lithiumtri(tert. butoxy)aluminiumhydrid in Tetrahydrofuran in Frage. Das Keton der Formel V kann auch z.B. mittels Diboran in Tetrahydrofuran oder mittels an sich bekannter katalytischer Hydrierung zur Verbindung der Formel I reduziert werden. Die Reaktionsbedingungen sind dem Fachmann aus analogen Reduktionen geläufig.

Die Verfahrensvariante c) wird durchgeführt, indem man das Keton der Formel V mit der Verbindung der Formel VI, zweckmässigerweise in einem inerten Verdünnungsmittel, vorzugsweise einem aprotischen Lösungsmittel, wie einem Aether oder einer ätheüartigen Verbindung, z.B. Diäthyläther Tetrahydrofuran oder Dioxan, und in einem Temperaturbereich zwischen -70°C und der Rückflusstemperatur des Reaktionsgemisches, vorzugsweise zwischen -30°C und der Raumtemperatur, umsetzt.

Die Umsetzung des Alkohols der Formel I' nach Verfahrensvariante d) wird vorzugsweise unter Verwendung von Phosphorpentachlorid, Thionylchlorid oder Phosphoroxychlorid als Chlorierungsmittel durchgeführt. Bei der Durchführung wird zweckmässigerweise in Gegenwart eines Verdünnungsmittels, insbesondere eines inerten aprotischen organischen Lösungsmittels, und gegebenenfalls auch in Gegenwart einer Base gearbeitet. Zu den bevorzugten Verdünnungsmitteln gehören aliphatische und aromatisch Kohlenwasserstoffe, wie n-Hexan, Benzol, Toluol und Xyole; halogenierte aliphatische Kohlenwasserstoffe, wie Methylenchlorid, Chloroform und Tetrachlorkohlenstoff; halogenierte aromatische Kohlenwasserstoffe, wie Chlorbenzol; und tertiäre Amine, wie Triäthylamin und Pyridin. Bevorzugte Basen sind Triäthylamin, Pyridin und Calciumcarbonat. Die Reaktionstemperaturen liegen im allgemeinen zwischen 0°C und der Rückflusstemperatur des Reaktionsgemisches, vorzugsweise zwischen der Raumtemperatur und der Rückflusstemperatur. Das Chlorierungsmittel wird vorzugsweise im Ueberschuss verwendet.

Auch die Umsetzung des Epoxids der Formel II nach Verfahrensvariante d) wird zweckmässigerweise in einem Ueberschuss an Chlorierungsmittel, z.B. Phosphorpentachlorid, und in Gegenwart eines Verdünnungsmittels durchgeführt. Als Verdünnungsmittel kommen insbesondere inerte aprotische organische Lösungsmittel, wie aliphatische und aromatische Kohlenwasserstoffe, z.B. n-Hexan, Benzol, Toluol und Xylole; und halogenierte aliphatische Kohlenwasserstoffe, z.B. Methylenchlorid, Chloroform und Tetrachlorkohlenstoff, in Frage. Vorzugsweise wird zwischen 20°C und der Rückflusstemperatur des Reaktionsgemisches gearbeitet.

Bei der Verfahrensvariante e) wird zweckmässigerweise der Thioäther der Formel VII zuerst mit einer starken Base, wie einem Alkalimetallmid, z.B. Lithiumiisopropylamid, in einem inerten Verdünnungsmittel, wie einem Kohlenwasserstoff oder einem Aether oder einer ätherartigen Verbindung, z.B. Tetrahydrofuran, und bei einer Temperatur von ca. -70°C behandelt. Anschliessend wird der Aldehyd bzw. das Keton der Formel VIII zugefügt und das Reaktionsgemisch auf Raumtemperatur gebracht. Auf diese Weise wird die Reaktion normalerweise innert kurzer Zeit beendet.

Bei der Verfahrensvariante f) handelt es sich um die N-Oxydation derjenigen Endprodukte der Formel I, worin $R^4$ die oben angegebenen Bedeutungen von $R^{4'}$ besitzt. Die Umsetzung kann zweckmässigerweise durchgeführt werden, indem man die Verbindung der Formel I" mittels Wasserstoffperoxid oder einer Persäure in Gegenwart eines inerten Verdünnungsmittels N-oxydiert.

Im Falle der Verwendung von Wasserstoffperoxid als Oxydationsmittel kommen als Verdünnungsmittel insbesondere niedere Alkanole, wie Methanol, Aethanol und Isopropanol, in Frage, und die N-Oxydation wird vorzugsweise in einem Temperaturbereich zwischen 0° und 60°C, insbesondere zwischen 20° und 40°C, vorgenommen.

Als Persäuren kommen vorzugsweise Peressigsäure, Perbenzoesäure und m-Chlorperbenzoesäure in Frage, wobei bevorzugt in einem halogenierten Kohlenwasserstoff, z.B. Methylenchlorid oder Chloroform, als Verdünnungsmittel gearbeitet wird. Die N-Oxydation mit einer Persäure erfolgt vorzugsweise in einem Temperaturbereich zwischen 0°C und der Rückflusstemperatur des Reaktionsgemisches, insbesondere zwischen 0°C und der Raumtemperatur. Eine besonders bevorzugte Ausführungsform dieses Verfahrens besteht darin, dass man die N-Oxydation mit m-Chlorperbenzoesäure in Chloroform in einem Temperaturbereich zwischen 0°C und der Raumtemperatur durchführt.

Zur Herstellung der Säureadditionssalze der Verbindungen der Formel I werden die Verbindungen I mit den gewunschten Säuren auf übliche Weise umgesetzt.

Die Isolierung und Reinigung der so hergestellten Verbindungen der Formel I bzw. der Säureadditionssalze können nach an sich bekannten Methoden erfolgen.

Die als Ausgangsmaterialien verwendbaren Epoxide der allgemeinen Formel II sind neu. Sie können hergestellt werden, indem man ein Halogenid der allgemeinen Formel

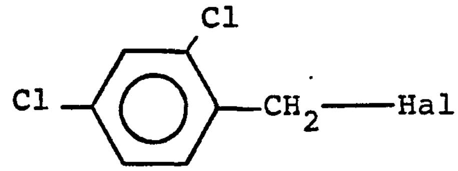

IX

worin Hal Chlor, Brom oder Jod bedeutet, mit einem Aldehyd bzw. Keton der allgemeinen Formel VIII, wie oben definiert, und mit Dimethylsulfid in wässrigem Medium umsetzt.

Diese Umsetzung wird zweckmässigerweise als Eintopfverfahren in Gegenwart eines inerten organischen Lösungsmittels und einer Base durchgeführt. Vorzugsweise wird deshalb in einem wässrig-organischen System gearbeitet. Als organische Lösungsmittel eignen sich insbesondere aliphatische und aromatische Kohlenwasserstoffe, wie n-Hexan, Benzol und Toluol; Alkohole, wie Methanol, Aethanol und Isopropanol; und Aether und ätherartige Verbindungen, wie Diäthyläther, Tetrahydrofuran und Dioxan. Als Basen sind wasserlösliche Alkalihydroxide, wie Natriumhydroxid und Kaliumhydroxid, bevorzugt. Es wird im allgemeinen zwischen der Raumtemperatur und der Rückflusstemperatur des Reaktionsgemisches gearbeitet.

Bei den Ausgangsmaterialien der allgemeinen Formel III handelt es sich zum Teil um diejenigen Verbindungen der Formel I, worin $R^1$ $OSO_2R^5$, $R^2$ Hydroxy und $R^4$ 3-Pyridyl oder 2-Pyrazinyl bedeuten. Solche Verbindungen können z.B.durch Umsetzung eines Epoxids der Formel II mit einer Alkansulfonsäure bzw. einer aromatischen Sulfonsäure gemäss der bzw. analog zur Verfahrensvariante a) hergestellt werden.

Die Ausgangsmaterialien der allgemeinen Formel V sind neu und können hergestellt werden, indem man ein Keton der allgemeinen Formel

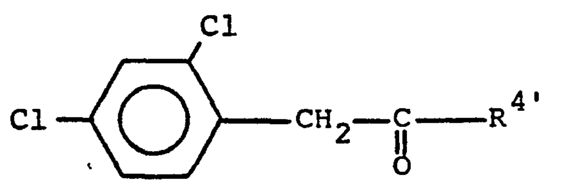

X

worin $R^4$ die oben angegebene Bedeutung besitzt, mit einem Alkylierungsmittel der allgemeinen Formel

$$R^{1''}—U \qquad XI$$

worin $R^{1''}$ die oben angegebene Bedeutung besitzt
und U eine Abgangsgruppe, insbesondere Chlor, Brom oder Jod, bedeutet,
behandelt.

Bei der Umsetzung wird mit Vorteil das Keton der Formel X mit einer Base wie Natriumhydrid oder Lithiumdiisopropylamid in einem Verdünnungsmittel, vorzugsweise einem aprotischen organischen Lösungsmittel, wie Tetrahydrofuran, Dimethoxyäthan oder Dimethylformamid, bei Reaktionstemperaturen zwischen -70°C und 50°C in ein Anion übergeführt, welches dann mit dem Alkylierungsmittel der Formel XI versetzt wird.

Die Ausgangsmaterialien der allgemeinen Formel VI sind entweder bekannt oder können nach an sich bekannten Methoden hergestellt werden.

Bei den Ausgangsmaterialien der allgemeinen Formel I' handelt es sich um diejenigen Verbindungen der Formel I, worin $R^2$ Hydroxy und $R^4$ 3-Pyridyl oder 2-Pyrazinyl bedeuten. Diese Verbindungen können z.B. gemäss bzw. analog zu den Verfahrensvarianten a) bis c) und e) hergestellt werden.

Die Ausgangsmaterialien der allgemeinen Formeln VII, VIII, IX und XI sind entweder bekannt oder können nach an sich bekannten Methoden hergestellt werden.

Die als Ausgangsmaterialien dienenden Ketone der allgemeinen Formel X sind entweder bekannt oder können nach an sich bekannten Methoden hergestellt werden (siehe z.B. die deutsche Offenlegungsschrift Nr. 2 221 546). Besonders bevorzugt ist jedoch das folgende Verfahren:

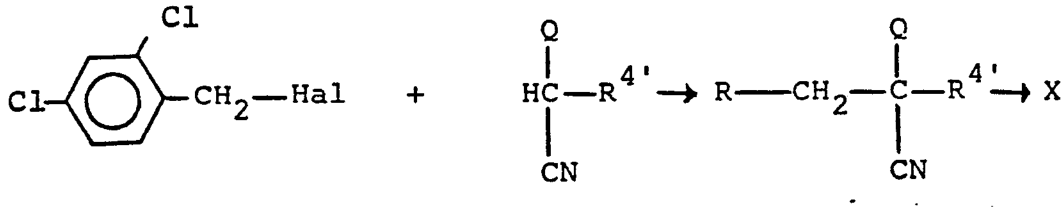

IX XII XIII

worin $R^{4'}$ und Hal die oben angegebenen Bedeutungen besitzen und Q einen disubstituierten Aminorest, z.B. Dimethylamino, Diäthylamino, Piperidino oder Morpholino, bedeutet.

Die Umsetzung der Verbindung der Formel IX mit der Verbindung der Formel XII kann z.B. unter den Bedingungen der Phasentransferkatalyse (siehe z.B. J. Dockx, Synthesis (1973), 441) bzw. unter Verwendung einer Base, wie z.B. Natriumhydrid oder Lithiumdiisopropylamid, in einem inerten Lösungsmittel, wie z.B. einem Aether oder einer ätherartigen Verbindung, z.B Tetrahydrofurn oder Dimethoxyäthan, in einem Temperaturbereich zwischen -70° C und 50° C vorzugsweise zwischen -30° C und der Raumtemperatur und unter Ausschluss von Wasser durchgeführt werden. Das so erhaltene Produkt der Formel XIII lässt sich hierauf durch Hydrolyse, z.B. durch konventionelle Behandlung mit einer wässrigen Säure, in das Keton der Formel X überführen. Als Säuren kommen für diesen Zweck insbesondere starke anorganische Säuren, wie Schwefelsäure, Chlorwasserstoffsäure und Bromwasserstoffsäure, sowie Sulfonsäuren, wie Benzolsulfonsäure und p-Toluolsulfonsäure, in Frage. Die Hydrolyse wird zweckmässigerweise in einem Temperaturbereich zwischen 20° C und der Rückflusstemperatur des Reaktionsgemisches, vorzugsweise zwischen 80° C und 100° C, ausgeführt.

Die erfindungsgemässen Verbindungen, d.h. die Verbindungen der Formel I und deren Säureadditionssalze, besitzen fungizide Wirkung und können dementsprechend zur Bekämpfung von Fungi in der Landwirtschaft und im Gartenbau Verwendung finden. Sie eignen sich insbesondere zur Eindämmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie im Erdboden und sind besonders wirksam bei der Bekämpfung von Botrytis cinerea (Graufäule); von echten Mehltaupilzen, wie beispielsweise Uncinula necator (Rebenmehltau), Erysiphe cichoracearum (Gurkenmehltau), Podosphaera leucotricha (Apfelmehltau) und Erysiphe graminis (Gerstenmehltau); von Venturia inaequalis (Apfelschorf); vom Helminthosporium oryzae (Braunfleckenkramkheit von Reis); und von Schadpilzen der Gattungen Puccinia, Uromyces, Hemileia, Rhizoctonia, Pemicillium, Septoria, Corticium und Cercospora.

Einzelne Vertreter der erfindungsgemässen Verbindungen besitzen zudem eine ausgeprägte Wirkung gegen holzzerstörende Pilze, wie z.B. Coniophora puteana und Gloeophyllum trabeum.

Die erfindumgsgemässen Verbindungen zeichnen sich durch lokale und bzw. oder systemische Wirkung aus.

Die erfindungsgemässen Verbindungen wirken unter Gewächshausbedingungen bereits bei einer Konzentration von 1 mg bis 500 mg Wirksubstanz pro Liter Spritzbrühe. Im Freiland werden vorteilhaft Konzentrationen von 25 g bis 1000 g Wirkstoff der Formel I pro Hektar und Behandlung zur Anwendung gebracht. Zur Bekampfung von samenbürtigen Pilzen im Beizverfahren werden mit Vorteil 0,05 g bis 1 5 g Wirkstoff der Formel I pro kg Samen verwendet.

Auch die oben erwähnten Materialien der allgemeinen Formel V sowie ihre N-Oxde sind als Fungizide wertvoll, da sie ein ähnliches Aktivitätsspektrum wie die Verbindungen der Formel I aufweisen. Diese Materialien können dementsprechend auch zur Bekämpfung von Fungi in der Landwirtschaft und im Gartenbau eingesetzt werden, und zwar auf gleiche Weise wie die Verbindungen der Formel I. Die N-Oxide können durch N-Oxydation der Verbindungen der Formel V hergestellt werden, und zwar analog zur Verfahrensvariante f).

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässem fungiziden Mittel sind dadurch gekennzeichnet, dass sie eine wirksame Menge mindestens einer Verbimdung der allgemeinem Formel I, wie oben definiert, oder vom eimem Säureadditiomssalz einer solchen Verbindumg sowie Formulierungshilfsstoffe enthaltem. Die Mittel enthalten zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Alumimiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chlor-5 äthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester: Ketone, wie Aceton, Methyläthylketon, Methylisobutylketon und Cyclohexanon; und stark polare Lösungs- bzw' Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere säure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol: UV-Absorber z.B. substituierte Benzophenone, Diphenylacrylnitrilsäureester und Zimtsäureester: und Deaktivatoren z.B. Salze der Aethylendiaminotetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u.a. gebrauchsfertige Lösungen, Emulsionen und Suspensionen, die sich beispielsweise als Spritzbrühen eignen. In solchen Spritzbrühen können z.B. Konzentrationen zwischen 0,0001 und 20 Gewichtsprozent vorliegen. In Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder suspendieren in geeigneten Lösungs- bzw. Dispersionsmittelm, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem fetten Trägerstoff vermischt werden z.B. durch Zusammenmahlen; oder man kann den festen Trägerstoff mit einer Lösung oder suspension des

Wirkstoffs imprägnieren und dann die Lösungs- oder Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch kann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werdem. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

**Herstellung der Wirkstoffe der Formel I:**

**Beispiel 1**

14 g cis-1-(2,4-Dichlorphenyl)-2-(3-pyridyl)-1,2-epoxypropan werden bei 0-5°C unter Rühren in 35 ml konzentrierter Salzsäure gelöst. Die Lösung wird nun 4 Stunden in diesem Temperaturbereich weitergerührt und anschliessend auf Eiswasser gegossen. Die entstandenen Kristalle werden abfiltriert, mit Wasser und danach mit Diäthyläther gewaschen und getrocknet. Nach Umkristallisation des so erhaltenen Rohproduktes (14 g Trockengewicht) wird es aus Methylenchlorid/Diäthyläther umkristallisiert und schliesslich mit Diäthyläther gewaschen. Man erhält 9,4 g α-(α,2,4-Trichlor-benzyl)-α-methyl-3-pyridylmethanol in Form des threo-Isomeren, Smp. 148-149,5°C.

Auf analoge Weise erhält man:

Aus cis 1-(2,4-Dichlorphenyl)-2-(3-pyridyl)-1,2-epoxypropan und konzentrierter Bromwasserstoffsäure das α-(α-Brom-2,4-dichlorbenzyl)-α-methyl-3-pyridylmethanol in Form des threo-Isomeren, Smp. 151°C (unter Zersetzung).

Aus 1-(2,4-Dichlorphenyl)-2-(3-pyridyl)-1,2-epoxypropan und Methanol unter Zugabe von konzentrierter Schwefelsäure das α-(2,4-Dichlor-α-methoxybenzyl)-α-methyl-3-pyridylmethanol als Diastereomerengemisch, Smp. 109-113°C.

Aus 1-(2,4-Dichlorphenyl)-2-(3-pyridyl)-1,2-epoxypropan und Methansulfonsäure in Methylenchlorid den Methansulfon-säure-2,4-Dichlor-α-[1-hydroxy-1-(3-pyridyl)-äthyl)-benzylester als Diastereomerengemisch, Smp. 137-141°C. Durch Chromatographie an Kieselgel erhält man zwei Isomere, Smp. 141-143°C bzw. 159-162°C.

Aus 1-(2,4-Dichlorphenyl)-2-(2-pyrazinyl)-1,2-epoxypropan und Methansulfonsäure den Methansulfonsäure-2,4-di-chlor-α-(1-hydroxy-1-(2-pyrazinyl)-äthyl)-benzylester als hochviskoses Oel.

**Beispiel 2**

168,5 g cis-1-(2,4-Dichlorphenyl)-2-(3-pyridyl)-1,2-epoxypropan in 160 ml Methylenchlorid werden mit 150 ml Methansulfonsäure versetzt, und das Reaktionsgemisch wird 5 Stunden bei 45°C sieden gelassen. Nach Abkühlen gibt man zum Gemisch 130 g Ammoniumchlorid und 900 ml konzentrierte Salzsäure und rührt dann 115 Stunden bei Raumtemperatur. Nun destilliert man mittels Wasserstrahlvacuum die Salzsäure ab, löst den Rückstand in Wasser und neutralisiert die entstandene Lösung mit 300 g Natriumcarbonat. Man extrahiert mit Aethylacetat, trocknet die organische Phase über wasserfreiem Magnesiumsulfat und kristallisiert nach Abdampfen des Lösungsmittels das Rohprodukt (230 g lösungsmittelfeucht; ca. 60% erythro-Isomeres und 40% threo-Isomeres) aus Toluol (fraktionierte Kristallisation). Das Produkt besteht aus α-(α,2,4-Trichlorbenzyl)-α-methyl-3-pyridylmethanol als erythro-Isomeres, Smp. 135-137°C (65 g), threo-Isomeres, Smp. 148-149,5°C (24 g) und Diastereomerengemisch (ca. 1:1- 85 g).

Auf analoge Weise erhält man:

Aus 1-(2,4-Dichlorphenyl)-2-(3-pyridyl)-1,2-epoxybutan, Methansulfonsäure und konzentrierter Salzsäure das α-(α,2,4-Trichlorbenzyl)-α-äthyl-3-pyridylmethanol als Diastereomerengemisch. Durch Chromatographie an Kieselgel erhält man ein reineres Produkt, Smp. 162-164°C.

Aus 1-(2,4-Dichlorphenyl)-2-(2-pyrazinyl)-1,2-epoxypropan, Methansulfonsäure und konzentrierter Salzsäure das α-(α, 2,4-Trichlorbenzyl)-α-methyl-2-pyrazinylmethanol als Diastereomerengemisch. Durch Chromatographie an Kieselgel erhält man zwei Isomere, das eine mit Smp. 133-134°C, das andere als hochviskoses Oel.

Aus 1-(2,4-Dichlorphenyl)-2-(2-pyrazinyl)-1,2-epoxypropan, Methansulfonsäure und konzentrierter Bromwasserstoffsäure das α-(α-Brom-2,4-dichlorbenzyl)-α-methyl-2-pyrazinylmethanol als Diastereomerengemisch. Durch Chromatographie an Kieselgel erhält man zwei Isomere, das eine mit Smp. 116-117°C, das andere mit Smp. 135-136°C.

Aus 1-(2,4-Dichlorphenyl)-2-(2-pyrazinyl)-1,2-epoxy-propan, Methansulfonsäure und konzentrierter Jodwasserstoffsäure das α-Jod-2,4-dichlorbenzyl)-α-methyl-2-pyrazinylmethanol, welches in zwei Diastereomere aufgetrennt wird das eine mit Smp. 183-185°C, das andere mit Smp. 152-153°C.

Aus cis-1-(2,4-Dichlorphenyl)-2-(3-pyridyl)-1,2-epoxypropan, Methansulfonsäure und konzentrierter Bromwasserstoffsäure das α-(α-Brom-2,4-dichlorbenzyl)-α-methyl-3-pyridylmethanol als Diastereomerengemisch. Durch Chromatographie an Kieselgel erhält man das threo-Isomere, Smp. 154°C (unter Zersetzung), und das erythro-Isomere, Smp. 135-136°C.

**Beispiel 3**

2,5 g 2-(2,4-Dichlorphenyl)-l-(3-pyridyl)-1-propanon werden in 10 ml Methanol gelöst und mit 0,4 g Natriumborhydrid versetzt. Nach dreistündiger Reaktionszeit bei Raumtemperatur wird das Reaktionsgemisch auf Eiswasser gegossen, und das Ganze wird mit Diäthyläther extrahiert. Die organische Phase wird gewaschen, über wasserfreiem Natriumsulfat getrocknet und das Lösungsmittel unter vermindertem Druck entfernt. Nach Kristallisation des Rückstandes aus Aethylacetat/n-Hexan erhält man α-(2,4-Dichlor-α-methylbenzyl)-3-pyridylmethanol, Smp. 94-96°C.

Auf analoge Weise erhält man durch Reduktion von 2-(2,4-Dichlorphenyl)-1-(3-pyridyl)-1-butanon unter Verwendung von Natriumborhydrid als Reduktionsmittel das α-(2,4-Dichlor-α-äthylbenzyl)-3-pyridylmethanol als zähes Oel.

**Beispiel 4**

Zum Grignardreagens aus 0,26 g Magnesiumspänen und 1,5 g Methyljodid in 15 ml Diäthyläther wird bei Raumtemperatur eine Lösung von 2 g 2-(2,4-Dichlorphenyl)-1-(3-pyridyl)-1-propanon in 10 ml Diäthyläther zugetropft. Nach einstündiger Reaktionszeit bei Raumtemperatur wird das Reaktionsgemisch in Wasser gegossen, und das Ganze wird mit Diäthyläther extrahiert. Die organische Phase wird gewaschen, über wasserfreiem Natriumsulfat getrocknet und das Lösungsmittel unter vermindertem Druck entfernt. Man erhält α-(2,4-Dichlor-α-methylbenzyl)-α-methyl-3-pyridylmethanol, Smp. 111-113°C.

Auf analoge Weise erhält man:

Aus Aethylmagnesiumbromid und 2-(2,4-Dichlorphenyl)-1-(3-pyridyl)-1-propanon das α-(2,4-Dichlor-α-methy1benzyl)-α-äthyl-3-pyridylmethanol als zähes Oel.

Aus Methylmagnesiumjodid und 2-(2,4-Dichlorphenyl)-1-(3-pyridyl)-1-butanon das α-(2,4-Dichlor-α-äthylbenzyl)-α-methyl-3-pyridylmethanol, Smp. 55-56°C.

**Beispiel 5**

11,2 g cis-1-(2,4-Dichlorphenyl)-2-(3-pyridyl)-1,2-epoxypropan werden in 40 ml absolutem Toluol gelöst. Die Lösung wird unter Rühren mit 17 g Phosphorpentachlorid versetzt, 16 Stunden stehen gelassen und danach weitere 45 Minuten bei 85°C gerührt. Nach Abkühlen des Reaktionsgemisches wird dies auf Eiswasser/Methylenchlorid gegossen. Das entstandene zweiphasige Reaktionsgemisch wird mit 70 g Natriumbicarbonat neutralisiert, die organische Phase abgetrennt und die wässrige Phase mit Methylenchlorid extrahiert. Anschliessend werden die vereinigten organischen Lösungen über wasserfreiem Natriumsulfat getrocknet und hierauf eingeengt. Durch Chromatographie des Rückstandes an Kieselgel (Laufmittel: Diäthyläther/Cyclohexan) und Kristallisation aus n-Pentan erhält man 9,2 g α-(α,2,4-Trichlorbenzyl)-α-methyl-3-pyridylmethylchlorid als 3:1-Diastereomerengemisch, Smp. 55-65°C. Durch Chromatographie an Kieselgel mit Toluol/Essigsäure (4:1) erhält man zwei Isomere, Smp. 68,5-69°C bzw. 71-73°C.

Auf analoge Weise erhält man durch ChloriErung vcn 1-(2,4-Dichlorphenyl)-2-(2-pyrazinyl)-1,2-epoxypropan mit Phosphorpentachlorid das α-(α,2,g-Trichlorbenzyl)-α-methyl-2-Pyrasinylmethylchlorid als Oel.

**Beispiel 6**

10 g α-(2 4-Dichlor-α-methylbenzy1)-3-pyridylmethanol werden in 25 ml Phosphoroxychlcrid während 90 Minuten bei 85°C gerührt. Dann wird auf Eis gegossen und durch Zugabe von Natriumbicarbonat neutralisiert. Das durch Extraktion mit Aethylacetat erhaltene Rohprodukt wird an Kieselgel chromatographiert. Mit n-Hexan/Aethylacetat (1:1) lässt sich 3-(α,2,4-Trichlor-ß-methylphenäthyl)-pyridin als Oel eluieren.

**Beispiel 7**

Zu einer Lösung von Lithiumdiisopropylamid (aus 1,6 g Diisopropylamin und einer äquivalenten Menge n-Butyllithium) in 10 ml Tetrahydrofuran bei -70°C wird 3 g 2,4-Dichlorbenzylmethylsulfid gegeben, und das Gemisch wird 1 Stunde bei dieser Temperatur reagieren gelassen. Dann fügt man 1,81 g Pyridin-3-carbaldehyd zu und lässt das Reaktionsgemisch sich langsam auf Raumtemperatur erwärmen. Es wird mit Wasser versetzt und mit Diäthyläther extrahiert. Das Reaktionsprodukt wird an Kieselgel mit Aethylacetat chromatographiert. Man erhält das α-(2,4-Dichlor-α-(methyl-thio)-benzyl]-3-pyridylmethanol als Diastereomerengemisch das nach einer Umkristallisation aus Aethylacetat/n-Hexan einen Smp. von 98-104°C aufweist.

Auf analoge Weise erhält man:

Aus 2,4-Dichlorbenzylmethylsulfid und 3-Acetylpyridin das α-[2,g-Dichlor-α-(methylthio)-benzyl]-α-methyl-3-pyridy1methanol, Smp. 130-136°C.

Aus 2,4-Dichlorbenzylmethylsulfid und 2-Acetylpyrazin das α-[2,4-Dichlor-α-(methylthio)-benzyl]-α-methyl-2-pyrazinylmethanol als Oel.

**Beispiel 8**

6 g α-(2,4-Dichlor-α-methylbenzyl)-3-pyridylmethanol werden in 200 ml Chloroform gelöst und bei 0°C mit 3,96 g 3-Chlorperbenzoesäure versetzt. Dann wird 16 Stunden bei ca. 4°C reagieren gelassen. Das Reaktionsgemisch wird auf 5%-ige Natriumcarbonatlösung gegossen und anschliessend mit Chloroform extrahiert. Beim Einengen der Lösung erhält man weisse Kristalle von α-(2,4-Dichlor-α-methylbenzyl)-3-pyridylmethanol-1-oxid, Smp. 167-170°C.

Auf analoge Weise erhält man aus α-(α,2,4,-Trichlor-benzyl)-α-methyl-3-pyridylmethanol und 3-Chlorperbenzoesäure das α-(α,2,4-Trichlorbenzyl)-α-methyl-3-pyridyl-methanol-1-oxid, Smp. 63-68°C.

**II Herstellung der Ausgangsmaterialien:**

**Beispiel 9**

Die in den Beispielen 1, 2 und 5 a1s Ausgangsmaterialien dienenden Epoxide der Formel II können wie folgt hergestellt werden: 140 g 2,4-Dichlorbenzylchlorid werden in 200 ml Wasser suspendiert und mit 60 ml Dimethylsulfid versetzt. Das Reaktionsgemisch wird nun während 20 Stunden bei einer Badtemperatur von 35-40°C gerührt.

Es wird ein Intensivkühler verwendet und mittels Methanol von -10°C gekühlt. Nach 20 Stunden Reaktionszeit gibt man zum gebildeten Sulfoniumsalz 200 ml 3-Acetyl-pyridin, 61 g Kaliumhydroxid in 100 ml Wasser und 200 ml n-Hexan zu. Die Suspension wird 2 Stunden bei 50-60°C Badtemperatur gerührt. Zur Aufarbeitung trennt man die wässrige Schicht ab. Die Hexanphase wird zweimal mit 100 ml Wasser ausgewaschen, über Wasserfreiem Natriumsulfat getrocknet und hierauf eingeengt. Der Rückstand wird säulenchromatographiert. Man erhält cis-1-(2,4-Dichlorphenyl)-2-(3-pyri-dyl)-1,2-epoxypropan, Smp. 68-70°C, und trans-1-(2,4-Dichlor-phenyl)-2-(3-pyridyl)-1,2-epoxypropan, Smp. 102-103°C.

Auf analoge Weise erhält man:

Aus 2,4-Dichlorbenzylchlorid, 3-Pyridinaldehyd und Dimethylsulfid das 1-(2,4-Dichlorphenyl)-2-(3-pyridyl)-1,2-epoxyäthan, Smp. 70-74°C.

Aus 2,4-Dichlorbenzylchlorid, 3-Propionylpyridin'und Dimethylsulfid das 1-(2,4-Dichlorphenyl)-2-(3-pyridyl)-1,2-epoxybutan, $n_D^{20}$ 1,5683.

Aus 2,4-Dichlorbenzylchlorid, 2-Acetylpyrazin und Dimethylsulfid das 1-(2,4-Dichlorphenyl)-2-(2-pyrazinyl)-1,2-epoxypropan, Smp. 107-109°C.

**Beispiel 10**

Die in den Beispielen 3 und 4 als Ausgangsmaterialien dienenden Ketone der Formel V können wie folgt hergestellt werden:

28 g 2,4-Dichlorbenzyl-3-pyridyl-keton werden in 800 ml Dimethylformamid gelöst und bei 0°C portionenweise mit 5,5 g Natriumhydrid (50%ige Dispersion in Oel) versetzt. Nach 2 Stunden bei Raumtemperatur wird 14,9 g Methyljodid zugefügt und weitere 3 Stunden bei Raumtemperatur gerührt. Dann wird auf Wasser gegossen, mit Diäthyläther trahiert, die organische Phase gewaschen und über wasserfreiem Natriumsulfat getrocknet und anschliessend eingeengt. durch Chromatographie an Kieselgel mit n-Hexan/Aethylacetat (1:2) erhält man das 2-(2,4-Dichlorphenyl)-1-(3-pyridyl)-1-propanon als Oel.

Auf analoge Weise erhält man:

Aus 2,4-Dichlorbenzyl-3-pyridyl-keton und Aethylbromid das 2-(2,4-Dichlorphenyl)-1-(3-pyridyl)-1-butanon als Oel.

Aus 2,4-Dichlorbenzyl-3-pyridyl-keton und Isopropylbromid das 2-(2,4-Dichlorphenyl)-3-methyl-1-(3-pyridyl)-1-butanon als Oel.

Das dabei als Ausgangsmaterial dienende 2,4-Dichlor-benzyl-3-pyridyl-keton kann wie folgt hergestellt werden:

Zu einem Gemisch von 700 g 50%-iger Natronlauge 23 g Tetrabutylammoniumjodid und 130 g α-(3-Pyridyl)-α-(4-morpho-lino)-acetonitril werden bei Raumtemperatur 125 g 2,4-Dichlorbenzylchlorid zugegeben. Nach 2 Stunden wird mit Diäthyläther extrahiert. Nach Abdampfen des Aethers bleibt als Rohprodukt α-(2,4-Dichlorbenzyl)-α-(3-pyridyl)-4-mor-pholino-acetonitril (Smp. 130-132°C) zurück. Dieses wird in 300 ml konzentrierte Salzsäure aufgenommen und die Lösung 12 Stunden auf Rückflusstemperatur erwärmt. Dann wird basisch gestellt, mit Aethylacetat extrahiert, die organische Phase gewaschen, über wasserfreiem Natriumsulfat getrocknet und eingeengt. Aus Aethylacetat/n-Hexan erhält man 2,4-Dichlorbenzyl-3-pyridyl-keton, Smp. 78-79°C.

**III Formulierungsbeispiele:**

**Beispiel 11**

1. Spritzpulver (für flüssige oder unter 75°C schmelzende Wirkstoffe)

| | Gewichtsteile |
|---|---|
| Wirkstoff der Formel I oder V | 50 |
| Hydratisierte Kieselsäure | 37 |
| Kaolin | 5 |
| Alkylphenoläthoxylat | 4 |
| Natrium-polynaphthalinsulfonat | 4 |
| | 100 |

Der flüssige oder geschmolzene Wirkstoff wird auf die Kieselgelsäure aufgezogen, die übrigen Komponenten zugemischt und das Ganze in einer geeigneten Mühle feingemahlen.

2. Spritzpulver (für feste, über 75°C schmelzende Wirkstoffe)

| | Gewichtsteile |
|---|---|
| Wirkstoff der Formel I oder V | 50 |
| Hydratisierte Kieselsäure | 5 |
| Kaolin | 42 |
| Natrium-laurylsulfat | 1 |
| Natrium-lignosulfonat | 2 |
| | 100 |

Die Komponenten werden miteinander vermischt und das Ganze in einer geeigneten Mühle feingemahlen.

**Beispiel 12**

Emulgierbares Konzentrat (für bei 20-25°C flüssige Wirkstoffe)

| | | Gewichtsteile |
|---|---|---|
| Wirkstoff der Formel I oder V | | 500 |
| Ricinusöl-äthoxylat | | 100 |
| Calcium-dodecylbenzolsulfonat | | 25 |
| Gemisch von $C_{10}$-Alkylbenzolen | ad | 1000 Vol.-Teile |

Die Komponenten werden miteinander vermischt, bis eine Klare Lösung entsteht.

**Patentansprüche**

für die Vertragsstaaten BE, CH, DE, FR, GB, IT, LI, NL

1. Verbindungen der allgemeinen Formel

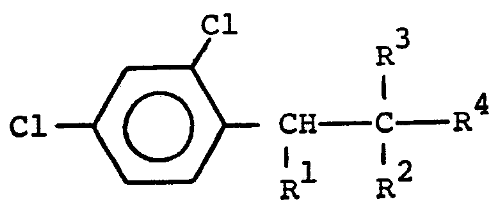

I

worin $R^1$ Halogen, $C_{1-4}$-Alkyl, $OR^5$, $SR^5$ oder, $OSO_2R^5$,
$R^2$ Hydroxy oder Chlor,
$R^3$ Wasserstoff oder $C_{1-4}$-Alkyl,
$R^4$ 3-Pyridyl, 3-Pyridyl-1-oxid, 2-Pyrazinyl, 2-Pyrazinyl-1-oxid, 2-Pyrazinyl-4-oxid oder 2-Pyrazinyl-1,4-dioxid und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann,
und deren Säureadditionssalze.

2. Verbindungen nach Anspruch 1, worin $R^4$ 3-Pyridyl oder 2-Pyrazinyl bedeutet.
3. Verbindungen nach Anspruch 1 oder 2, worin $R^3$ Wasserstoff oder Methyl bedeutet.
4. α-(α 2,4-Trichlorbenzyl)-α-methyl-3-pyridylmethanol.
5. α-(2,4-Dichlor-α-methylbenzyl)-3-pyridylmethanol.
6. α-(α-Brom-2,4-dichlorbenzyl)-α-methyl-2-pyrazinylmethanol.
7. α-(α-Brom-2,4-dichlorbenzyl)-α-methyl-3-pyridylmethanol.
8. Eine Verbindung nach Anspruch 2, ausgewählt aus:
α-(2,4-Dichlor-α-methoxybenzyl)-α-methyl-3-pyridylmethanol,
Methansulfonsäure-2,4-dichlor-α-[1-hydroxy-1-(3-pyridyl)-äthyl]-benzylester,
Methansulfonsäure-2,4-dichlor-α-[1-hydroxy-1-(2-pyrazinyl)-äthyl]-benzylester,
α-(α,2,4-Trichlorbenzyl)-α-äthyl-3-pyridylmethanol,
α-(α,2,4-Trichlorbenzyl)-α-methyl-2-pyrazinylmethanol,
α-(α-Jod-2,4-dichlorbenzyl)-α-methyl-2-pyrazinylmethanol,
α-(2,4-Dichlor-α-methylbenzyl)-α-methyl-3-pyridylmethanol,
α-(2,4-Dichlor-α-methylbenzyl)-α-äthyl-3-pyridylmethanol,
α-(2,4-Dichlor-α-äthylbenzyl)-α-methyl-3-pyridylmethanol,
α-(α,2,4-Trichlorbenzyl)-α-methyl-3-pyridylmethylchlorid,
α-[2,4-Dichlor-α-(methylthio)-benzyl]-3-pyridylmethanol,
α-(α,2,4-Trichlorbenzyl)-α-methyl-3-pyridylmethylchlorid,
α-[2,4-Dichlor-α-(methylthio)-benzyl]-α-methyl-3-pyridylmethanol und
α-[2,4-Dichlor-α-(methylthio)-benzyl]-α-methyl-2-pyrazinylmethanol.
9. Eine Verbindung nach Anspruch 1, ausgewählt aus:
α-(2,4-Dichlor-α-äthylbenzyl)-3-pyridylmethanol,
3-(α,2,4-Trichlor-β-methylphenäthyl)-pyridin,

α-(2,4-Dichlor-α-methylbenzyl)-3-pyridylmethanol-1-oxid und
α-(α,2,4-Tricholorbenzyl)-α-methyl-3-pyridylmethanol-1-oxid.

10. Verbindungen nach einem der Ansprüche 1 bis 9 als fungizide Wirkstoffe.

11. Fungizide Mittel, dadurch gekennzeichnet, dass es eine wirksame Menge mindestens einer Verbindung der allgemeinen Formel

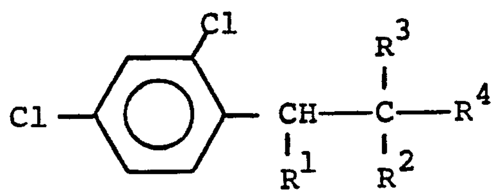

I

worin $R^1$ Halogen, $C_{1-4}$-Alkyl, $OR^5$, $SR^5$ oder $OSO_2R^5$,
$R^2$ Hydroxy oder Chlor,
$R^3$ Wasserstoff oder $C_{1-4}$-Alkyl,
$R^4$ 3-Pyridyl 3-Pyridyl-1-oxid, 2-Pyrazinyl, 2-Pyrazinyl-1-oxid, 2-Pyrazinyl-4-oxid oder 2-Pyrazinyl-1,4-dioxid und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann,
oder eines Säureadditionssalzes davon sowie Formulierungshilfsstoffe enthält.

12. Fungizides Mittel nach Anspruch 11, worin $R^4$ 3-Pyridyl oder 2-Pyrazinyl bedeutet.

13. Fungizides Mittel nach Anspruch 12, dadurch gekennzeichnet, dass es eine wirksame Menge α-(α,2,4-Trichlorbenzyl)-α-methyl-3-pyridylmethanol sowie Formulierungshilfsstoffe enthält.

14. Fungizides Mittel nach Anspruch 12, dadurch gekennzeichnet, dass es eine wirksame Menge α-(2,4-Diochlor-α-methylbenzyl)-3-pyridylmethanol sowie Formulierungshilfsstoffe enthält.

15. Fungizides Mittel nach Anspruch 12, dadurch gekennzeichnet, dass es eine wirksame Menge α-(α-Brom-2,4-dichlorbenzyl)-α-methyl-2-pyrazinylmethanol sowie Formulierungshilfsstoffe enthält.

16. Fungizides Mittel nach Anspruch 12, dadurch gekennzeichnet, dass es eine wirksame Menge α-(α-Brom-2,4-. dichlorbenzyl)-α-methyl-3-pyridylmethanol sowie Formulierungshilfsstoffe enthält.

17. Verfahren zur Herstellung von Verbindungen der allgemeinen Formel

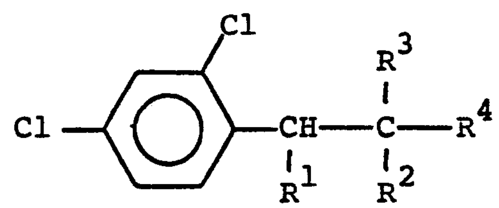

I

worin $R^1$ Hallogen, $C_{1-4}$-Alkyl, $0R^5$, $SR^5$ oder $OSO_2R^5$,
$R^2$ Hydroxy oder Chlor,
$R^3$ Wasserstoff oder $C_{1-4}$-Alkyl,
$R^4$ 3-Pyridyl, 3-Pyridyl-1-oxid, 2-Pyrazinyl, 2-Pyrazinyl-1-oxid, 2-Pyrazinyl-4-oxid oder 2-Pyrazinyl-1,4-dioxid und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann,
und von ihren Säureadditionssalzen, dadurch gekennzeichnet, dass man

a) zwecks Herstellung der Verbindungen der Formel I, worin $R^1n$ Halogen, $OR^5$, $SR^5$ oder $OSO_2R^5$, $R^2$ Hydroxy und $R^4$ 3-Pyridyl oder 2-Pyrazinyl bedeuten, ein Epoxid der allgemeinen Formel

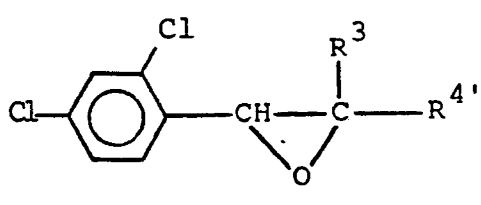

II

worin $R^3$ die oben angegebene Bedeutung besitzt
und $R^{4'}$ 3-Pyridyl oder 2-Pyrazinyl bedeutet, oder einen Alkohol der allgemeinen Formel

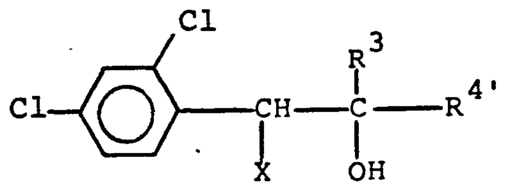

III

worin $R^3$ und $R^{4'}$ die oben angegebenen Bedeutungen besitzen und X einen Alkylsulfonatrest oder Arylsulfonatrest bedeutet, mit einer Verbindung der allgemeinen Formel

$$R^{1'}-H \qquad IV$$

worin $R^{1'}$ Halogen, $OR^5$, $SR^5$ oder $OSO_2R^5$ bedeutet und $R^5$ die oben angegebene Bedeutung besitzt, umsetzt,

b) zwecks Herstellung der Verbindungen der Formel I, worin $R^1$ $C_{1-4}$-Alkyl, $R^2$ Hydroxy, $R^3$ Wasserstoff und $R^4$ 3-Pyridyl oder 2-Pyrazinyl bedeuten, ein Keton der allgemeinen Formel

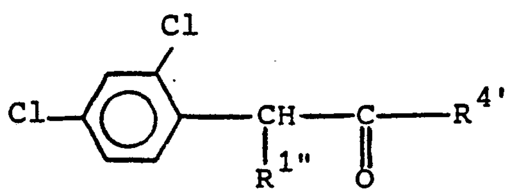

V

worin $R^{4'}$ die oben angegebene Bedeutung besitzt und $R^{1''}$ $C_{1-4}$-Alkyl bedeutet, reduziert,

c) zwecks Herstellung der Verbindungen der Formel I, worin $R^1$ $C_{1-4}$-Alkyl, $R^2$ Hydroxy, $R^3$ $C_{1-4}$-Alkyl und $R^4$ 3-Pyridyl oder 2-Pyrazinyl bedeuten, ein Keton der allgemeinen Formel V, wie oben definiert, mit einer Verbindung der allgemeinen Formel

$$R^{3'}-Y \qquad VI$$

worin $R^{3'}$ $C_{1-4}$-Alkyl, Y Lithium oder MgZ und Z Halogen bedeuten, umsetzt,

d) zwecks Herstellung der Verbindungen der Formel I, worin $R^2$ Chlor und $R^4$ 3-Pyridyl oder 2-Pyrazinyl bedeuten, einen Alkohol der allgemeinen Formel

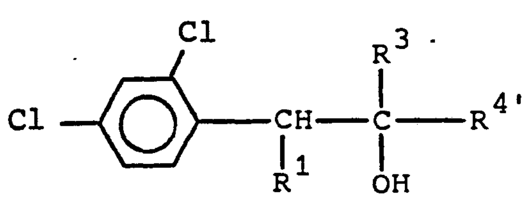

I'

worin $R^1$, $R^3$ und $R^{4'}$ die oben angegebenen Bedeutungen besitzen, oder ein Epoxid der allgemeinen Formel II, wie oben definiert, mit einem Chlorierungsmittel behandelt,

e) zwecks Herstellung der Verbindungen der Formel I, worin $R^1$ $SR^5$ und $R^2$ Hydroxy bedeuten, einen Thioäther der allgemeinen Formel

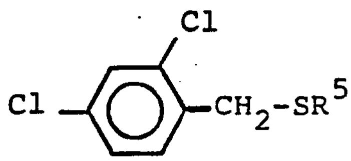

VII

worin $R^5$ die oben angegebene Bedeutung besitzt, mit einem Aldehyd bzw. Keton der allgemeinen Formel

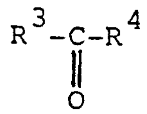

VIII

worin $R^3$ und $R^4$ die oben angegebenen Bedeutungen besitzen, umsetzt, oder

f) zwecks Herstellung der Verbindungen der Formel I, worin $R^4$ 3-Pyridyl-1-oxid, 2-Pyrazinyl-1-oxid, 2-Pyrazinyl-4-oxid oder 2-Pyrazinyl-1,4-dioxid bedeutet, ein Pyridin- bzw. Pyrazin-Derivat der allgemeinen Formel

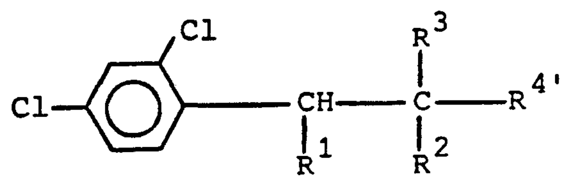

I"

worin $R^1$, $R^2$, $R^3$ unde $R^{4'}$ die oben angegebenen Bedeutungen besitzen, N-oxydiert und erwünschtenfalls eine erhaltene Verbindung der Formel I mit einer Säure zur Herstellung des entsprechenden Säureadditionssalzes umsetzt.

18. Verfahren nach Anspruch 17, dadurch gekennzeichnet, dass $R^4$ in der Formel I 3-Pyridyl oder 2-Pyrazinyl bedeutet und die Variante a), b), c) oder d) angewendet wird.

19. Verfahren zur Bekämpfung von Fungi in der Landwirtschaft und im Gartenbau, dadurch gekennzeichnet, dass man das zu schützende Gut mit einer wirksamen Menge mindestens einer der in den Ansprüchen 1 bis 9 genannten Verbindungen bzw. eines der in den Ansprüchen 11 bis 16 genannten Mittel behandelt.

20. Verwendung einer der in den Ansprüchen 1 bis 9. genannten Verbindungen bzw. eines der in den Ansprüchen 11 bis 16 genannten Mittel zur Bekämpfung von.Fungi.in der Landwirtschaft und im Gartenbau.

21. Verbindungen der allgemeinen Formel

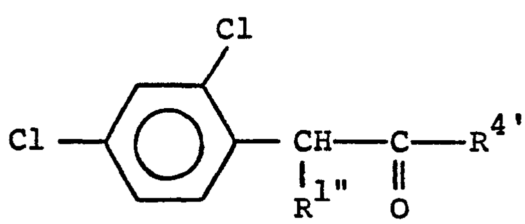

V

worin $R^{1''}$ $C_{1-4}$-Alkyl und $R^{4'}$ 3-Pyridyl oder 2-Pyrazinyl bedeuten, sowie ihre N-Oxide.

22. Fungizides Mittel, dadurch gekennzeichnet, dass es eine wirksame Menge mindestens einer Verbindung der allgemeinen Formel

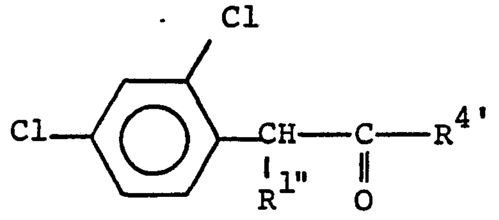

V

worin $R^{1''}$ $C_{1-4}$-Alkyl
und $R^{4'}$ 3-Pyridyl oder 2-Pyrazinyl bedeuten,
oder eines N-Oxides davon sowie Formulierungshilfsstoffe enhält.

**Patentansprüche**

für den Vertragsstaat AT

1. Fungizides Mittel, dadurch gekennzeichnet, dass es eine wirksame Menge mindestens einer Verbindung der allgemeinen Formel

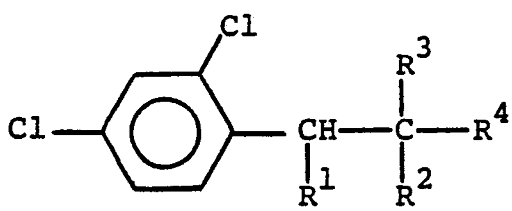

I

worin $R^1$ Halogen, $C_{1-4}$-Alkyl, $OR^5$, $SR^5$ oder $OSO_2R^5$,
$R^2$ Hydroxy oder Chlor,
$R^3$ Wasserstoff oder $C_{1-4}$-Alkyl,
$R^4$ 3-Pyridyl, 3-Pyridyl-1-oxid 2-Pyrazinyl, 2-Pyrazinyl-1-oxid, 2-Pyrazinyl-4-oxid oder 2-Pyrazinyl-1,4-dioxid
und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann,
oder eines Säureadditionssalzes davon sowie Formulierungshilfsstoffe enthält.

2. Fungizides Mittel nach Anspruch 1, worin $R^4$ 3-Pyridyl oder 2-Pyrazinyl bedeutet.

3. Fungizides Mittnl nach Anspruch I oder 2, worin $R^3$ Wasserstoff oder.Methyl bedeutet.

4. Fungizides Mittel nach Anspruch 2, dadurch gekennzeichnet, dass es eine wirksame Menge α-(α,2,4-Trichlor-benzyl)-α-methyl-3-pyridylmethanol sowie Formulierungshilfsstoffe enthält.

5. Fungizides Mittel nach Anspruch 2, dadurch gekennzeichnet, dass es eine wirksame Menge α-(2,4-Dichlor-α-methylbenzyl)-3-pyridylmethanol sowie Formulierungshilfsstoffe enthält.

6. Fungizides Mittel nach Anspruch 2, dadurch gekennzeichnet, dass es eine wirksame Menge α-(α-Brom-2,4-dichlorbenzyl)-α-methyl-2-pyrazinylmethanol sowie Formulierungshilfsstoffe enthält.

7. Fungizides Mittel nach Anspruch 2, dadurch gekennzeichnet, dass es eine wirksame Menge α-(α-Brom-2,4-dichlorbenzyl)-α-methyl-3-pyridylmethanol sowie Formulierungshilfsstoffe enthält.

8. Fungizides Mittel nach Anspruch 2, dadurch gekennzeichnet, dass es eine wirksame Menge einer Verbindung ausgewählt aus:
α-(2,4-Dichlor-α-methoxybenzyl)-α-methyl-3-pyridylmethanol,
Methansulfonsäure-2,4-dichlor-α-[1-hydroxy-1-(3-pyridyl)-äthyl]-benzylester,
Methansulfonsäure-2,4-dichlor-α-[1-hydroxy-1-(2-pyrazinyl)-äthyl]-benzylester,
α-(α,2,4-Trichlorbenzyl)-α-äthyl-3-pyridylmethanol,
α-(α,2,4-Trichlorbenzyl)-α-methyl-2-pyrazinylmethanol,
α-(α-Jod-2,4-dichlorbenzyl)-α-methyl-2-pyrazinylmethanol,
α-(2,4-Dichlor-α-methylbenzyl)-α-methyl-3-pyridylmethanol,
α-(2,4-Dichlor-α-methylbenzyl)-α-äthyl-3-pyridylmethanol,
α-(2,4-Dichlor-α-äthylbenzyl)-α-methyl-3-pyridylmethanol,
α-(α,2,4-Trichlorbenzyl)-α-methyl-3-pyridylmethylchlorid,
α-[2,4-Dichlor-α-(methylthio)-benzyl]-3-pyridylmethanol
α-(α,2,4-Trichlorbenzyl)-α-methyl-3-pyridylmethylchlorid
α-[2,4-Dichlor-α-(methylthio)-benzyl]-α-methyl-3-pyridylmethanol und
α-[2,4-Dichlor-α-(methylthio)-benzyl]-α-methyl-2-pyrazinylmethanol,
sowie Formulierungshilfsstoffe enthält.

9. Fungizides Mittel nach Anspruch 1, dadurch gekennzeichnet, dass es eine wirksame Menge einer Verbindung ausgewählt aus:
α-(2,4-Dichlor-α-äthylbenzyl)-3-pyridylmethanol,
3-(α,2,4-Trichlor-β-methylphenäthyl)-pyridin,
α-(2,4-Dichlor-α-methylbenzyl)-3-pyridylmethanol-1-oxid und
α-(α,2,4-Trichlorbenzyl)-α-methyl-3-pyridylmethanol-1-oxid,
sowie Formulierungshilfsstoffe enthält.

10. Verfahren zur Herstellung von Verbindungen der allgemeinen Formel

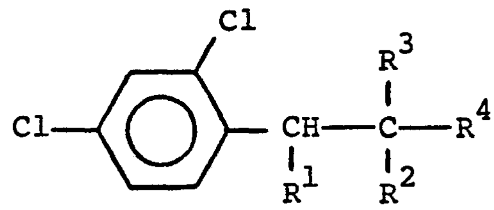
I

worin $R^1$ Halogen, $C_{1-4}$-Alkyl, $OR^5$, $SR^5$ oder $OSO_2R^5$,
$R^2$ Hydroxy oder Chlor,
$R^3$ Wasserstoff oder $C_{1-4}$-Alkyl,
$R^4$ 3-Pyridyl, 3-Pyridyl-1-oxid, 2-Pyrazinyl, 2-Pyrazinyl-1-oxid, 2-Pyrazinyl-4-oxid oder 2-Pyrazinyl-1,4-dioxid
und $R^5$ $C_{1-4}$-Alkyl bedeuten,
wobei, falls $R^2$ Chlor und $R^3$ $C_{1-4}$-Alkyl bedeuten,
$R^1$ nicht Brom oder Jod sein kann,
und von ihren Säureadditionssalzen, dadurch gekennzeichnet, dass man

a) zwecks Herstellung der Verbindungen der Formel I, worin $R^1$ Halogen, $OR^5$, $SR^5$ oder $OSO_2R^5$, $R^2$ Hydroxy und $R^4$ 3-Pyridyl oder 2-Pyrazinyl bedeuten, ein Epoxid der allgemeinen Formel

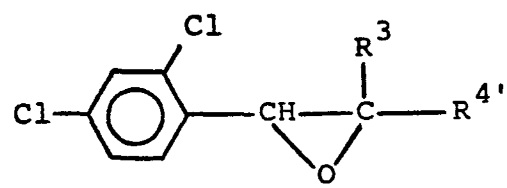
II

worin $R^3$ die oben angegebene Bedeutung besitzt
und $R^{4'}$ 3-Pyridyl oder 2-Pyrazinyl bedeutet,
oder einen Alkohol der allgemeinen Formel

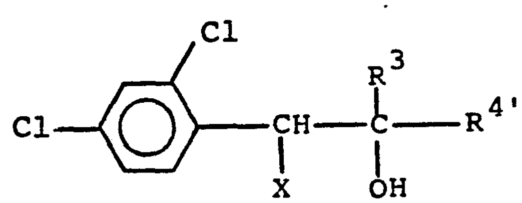
III.

worin $R^3$ und $R^{4'}$ die oben angegebenen Bedeutungen besitzen
und X einen Alkylsulfonatrest oder Arylsulfonatrest bedeutet,
mit einer Verbindung der allgemeinen Formel

$$R^{1'}—H \qquad IV$$

worin $R^{1'}$ Halogen, $OR^5$, $SR^5$ oder $OSO_2R^5$bedeutet
und $R^5$ die oben angegebene Bedeutung besitzt, umsetzt,

b) zwecks Herstellung der Verbindungen der Formel I, worin $R^1$ $C_{1-4}$-Alkyl, $R^2$ Hydroxy, $R^3$ Wasserstoff und $R^4$ 3-Pyridyl oder 2-Pyrazinyl bedeuten, ein Keton der allgemeinen Formel

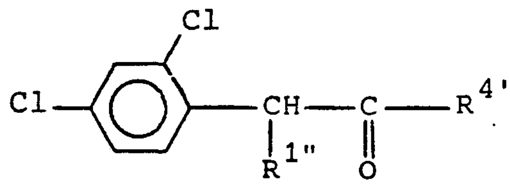

V

worin $R^{4'}$ die oben angegebene Bedeutung besitzt
und $R^{1''}$ $C_{1-4}$-Alkyl bedeutet, reduziert,

c) zwecks Herstellung der Verbindungen der Formel I, worin $R^1$ $C_{1-4}$-Alkyl, $R^2$ Hydroxy, $R^3$ $C_{1-4}$-Alkyl und $R^4$ 3-Pyridyl oder 2-Pyrazinyl bedeuten, ein Keton der allgemeinen Formel V, wie oben definiert, mit einer Verbindung der allgemeinen Formel

$R^{3'}$—Y VI

worin $R^{3'}$ $C_{1-4}$-Alkyl,
Y Lithium oder MgZ
und Z Halogen bedeuten, umsetzt,

d) zwecks Herstellung der Verbindungen der Formel I, worin $R_2$ Chlor und $R^4$ 3-Pyridyl oder 2-Pyrazinyl bedeuten, einen Alkohol der allgemeinen Formel

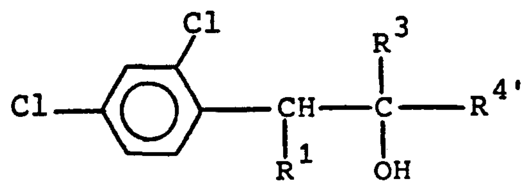

I'

worin $R^1$, und $R^4$ die oben angegebenen Bedeutungen besitzen,
oder ein Epoxid der allgemeinen Formel II, wie oben definiert, mit einem Chlorierungsmittel behandelt,

e) zwecks Herstellung der Verbindungen der Formel I, worin $R^1$ $SR^5$ und $R^2$ Hydroxy bedeuten, einen Thioäther der allgemeinen Formel

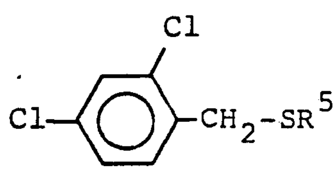

VII

worin $R^5$ die oben angegebene Bedeutung besitzt,
mit einem Aldehyd bzw. Keton der allgemeinen Formel

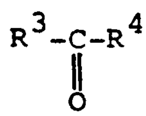

VIII

worin $R^5$ und $R^4$ die oben angegebenen Bedeutungen besitzen,
umsetzt, oder

f) zwecks Herstellung der Verbindungen der Formel I, worin $R^4$ 3-Pyridyl-1-oxid, 2-Pyrazinyl-1-oxid, 2-Pyrazinyl-4-oxid oder 2-Pyrazinyl-1,4-dioxid bedeutet, ein Pyridin- bzw. Pyrazin-Derivat der allgemeinen Formel

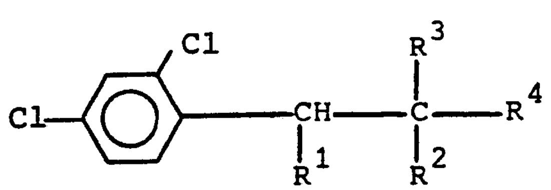

I''

worin $R^1$, $R^2$, $R^3$ und $R^{4'}$ die oben angegebenen Bedeutungen besitzen, N-oxydiert und erwünschtenfalls eine erhaltene Verbindung der Formel I mit einer Säure zur Herstellung des entsprechenden Säureadditionssalzes umsetzt.

11. Verfahren nach Anspruch 10, dadurch gekennzeichnet, dass $R^4$ in der Formel I 3-Pyridyl oder 2-Pyrazinyl bedeutet und die Variante a), b), c) oder d) angewendet wird.

12. Verfahren zur Herstellung eines fungiziden Mittels, dadurch gekennzeichnet, dass man mindestens eine der in den Ansprüchen 1 bis 9 genannten Verbindungen mit Formulierungshilfsstoffen vermischt.

13. Verfahren zur Bekämpfung von Fungi in der Landwirtschaft und im Gartenbau dadurch gekennzeichnet, dass man das zu schützende Gut mit einer wirksamen Menge einer bzw. eines der in den Ansprüchen 1 bis 9 genannten Verbindungen bzw. Mittel behandelt.

14. Verwendung einer bzw. eines der in den Ansprüchen 1 bis 9 genannten Verbindungen bzw. Mittel zur Bekämpfung von Fungi in der Landwirtschaft und im Gartenbau.

15. Fungizides Mittel, dadurch gekennzeichnet, dass es eine wirksame Menge mindestens einer Verbindung der allgemeinen Formel

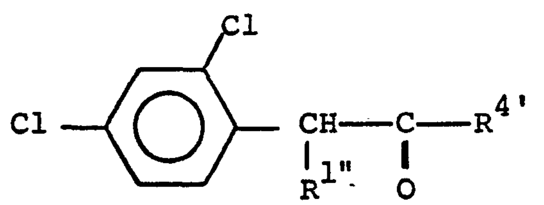

V

worin $R^{1''}$ $C_{1-4}$-Alkyl und $R^{4'}$ 3-Pyridyl oder 2-Pyrazinyl bedeuten, oder eines N-Oxides davon sowie Formulierungshilfsstoffe enthält.

**Claims**

for the Contracting States BE, CH, DE,FR, GB, IT, LI, NL

1. Compounds of the general formula

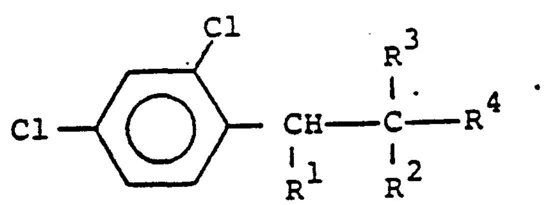

I

wherein $R^1$ signifies halogen, $C_{14}$-alkyl, $OR^5$, $SR^5$ or $OSO_2R^5$,
$R^2$ signifies hydroxy or chlorine.
$R^3$ signifies hydrogen or $C_{1-4}$-alkyl,
$R^4$ signifies 3-pyridyl, 3-pyridyl 1-oxide, 2-pyrazinyl, 2-pyrazinyl 1-oxide, 2-pyrazinyl 4-oxide or 2-pyrazinyl 1,4-dioxide
and $R^5$ signifies $C_{1-4}$-alkyl, whereby $R^1$ can not be bromine or iodine where $R^2$ signifies chlorine and $R^3$ signifies $C_{1-4}$-alkyl, and their acid addition salts.

2. Compounds according to claim I, wherein $R^4$ signifies 3-pyridyl or 2-pyrazinyl.

3. Compounds according to claim 1 or 2, wherein $R^3$ signifies hydrogen or methyl.

4. α-(α,2,4-Trichlorobenzyl)-α-methyl-3-pyridylmethanol.

5. α-(2,4-Dichloro-α-methylbenzyl)-3-pyridylmethanol.

6. α-(α-Bromo-2,4-dichlorobenzyl)-α-methyl-2-pyrazinylmethanol.

7. α-(α-Bromo-2,4-dichlorobenzyl)-α-methyl-3-pyridylmethanol.

8. A compound according to claim 2, selected from:
α-(2,4-Dichloro-α-methoxybenzyl)-α-methyl-3-pyridylmethanol,
2,4-dichloro-α-[l-hydroxy-l-(3-pyridyl)-ethyl]-benzyl methanesulphonate,
2,4-dichloro-α-[l-hydroxy-l-(2-pyrazinyl)-ethyl]-benzyl methanesulphonate,
α-(α,2,4-trichlorobenzyl)-α-ethy1-3-pyridylmethanol,
α-(α,2,4-trichlorobenzyl)-α-methyl-2-pyrazinylmethanol,
α-(α-iodo-2,4-dich1orobenzyl)-α-methyl-2-pyrazinylmethanol,

α-(2,4-dichloro-α-methylbenzyl)-α-methyl-3-pyridylmethanol,
α-(2,4-dichloro-α-methylbenzyl)-α-ethyl-3-pyridylmethanol,
α-(2,4-dichloro-α-ethylbenzyl)-α-methyl-3-pyridylmethanol,
α-(α,2,4-trichlorobenzyl)-α-methyl-3-pyridylmethyl chloride,
α-[2,4-dichloro-α-(methylthio)-benzyl]-3-pyridylmethanol,
α-(α,2,4-trichlorobenzyl)-α-methyl-3-pyridylmethyl chloride,
α-[2,4-dichloro-α-(methylthio)-benzyl]-α-methyl-3-pyridylmethanol and
α-[2,4-dichloro-α-(methylthio)-benzyl]-α-methyl-2-pyrazinylmethanol.

9. A compound according to claim 1 selected from:
α-(2.4-Dichloro-α-ethylbenzyl)-3-pyridylmethanol.
3-(α,2,4-trichloro-ß-methylphenethyl)pyridine,
α-(2,4-dichloro-α-methylbenzyl)-3-pyridylmethanol 1-oxide and
α-(α,2,4-trichlorobenzyl)-α-methyl-3-pyridylmethanol 1-oxide.

10. Compounds according to any one of claims 1 to 9 as fungicidal active substances.

11. A fungicidal composition, characterized in that it contains an effective amount of at least one compound of the general formula

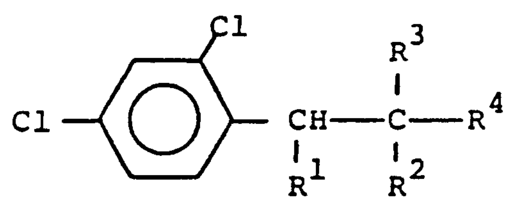

I

wherein $R^1$ signifies halogen, $C_{1-4}$-alkyl, $OR^5$, $SR^5$ or $OSO_2R^5$,
$R^2$ signifies hydroxy or chlorine,
$R^3$ signifies hydrogen or $C_{1-4}$-alkyl,
$R^4$ signifies 3-pyridyl, 3-pyridyl 1-oxide,
2-pyrazinyl, 2-pyrazinyl l-oxide, 2-pyrazinyl 4-oxide or 2-pyrazinyl 1,4-dioxide
and $R^5$ signifies $C_{1\ 4}$-alkyl,
whereby R can not be bromine or iodine where $R^2$ signifies chlorine and R signifies $C_{1\ 4}$-alkyl,
or an acid addition salt thereof as well as formulation adjuvants.

12. A fungicidal composition according to claim 11, wherein R signifies 3-pyridyl or 2-pyrazinyl.

13. A fungicidal composition according to claim 12, characterized in that it contains an effective amount of α-(α,2,4-trichlorobenzyl)-α-methyl-3-pyridylmethanol as well as formulation adjuvants.

14. A fungicidal composition according to claim 12, characterized in that it contains an effective amount of α-(2,4-dichloro-α-methylbenzyl)-3-pyridylmethanol as well as formulation adjuvants.

15. A fungicidal composition according to claim 12, characterized in that it contains an effective amount of α-(α-bromo-2,4-dichlorobenzyl)-α-methyl-2-pyrazinylmethanol as well as formulation adjuvants.

16. A fungicidal composition according to claim 12, characterized in that it contains an effective amount of α-(α-bromo-2,4-dichlorobenzyl)-α-methyl-3-pyridyl-methanol as well as formulation adjuvants.

17. A process for the manufacture of compounds of the general formula

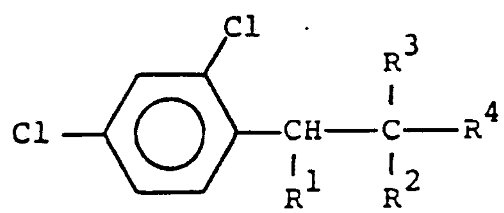

I

wherein $R^1$ signifies halogen, $C_{1-4}$-alkyl, $OR^5$, $SR^5$ or $OSO_2R^5$,
$R^2$ signifies hydroxy or chlorine,
$R^3$ signifies hydrogen or $C_{1-4}$-alkyl,
$R^4$ signifies 3-pyridyl, 3-pyridyl 1-oxide 2-pyrazinyl, 2-pyrazinyl 1-oxide, 2-pyrazinyl 4-oxide or 2-pyrazinyl 1,4-dioxide
and $R^5$ signifies $C_{1-4}$-alkyl,
whereby $R^1$ can not be bromine or iodine where $R^2$ signifies chlorine and $R^3$ signifies $C_{1\ 4}$-alkyl,
and of their acid addition salts, characterized by

a) for the manufacture of the compounds of formula I in which $R^1$ signifies halogen, OR, SR or $OSO_2R$, $R^2$ signifies hydroxy and R signifies 3-pyridyl or 2-pyrazinyl, reacting an epoxide of the general formula

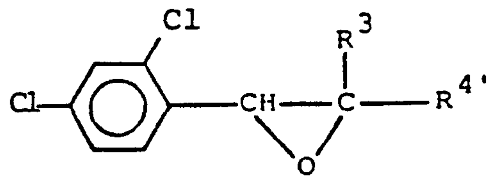

II

wherein $R^3$ has the significance given above and $R^4$ signifies 3-pyridyl or 2-pyrazinyl, or an alcohol of the general formula

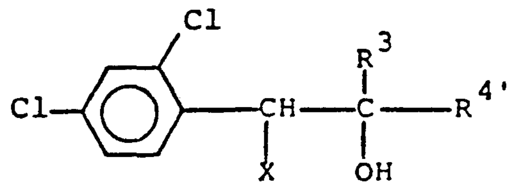

III

wherein $R^3$ and $R^4$ have the significances given above and X signifies an alkylsulphonate residue or arylsulphonate residue, with a compound of the general formula $R^{1'}-H$

$$R^{1'}-H \qquad IV$$

wherein $R^{1'}$ signifies halogen, $OR^5$, $SR^5$ or $OSO_2R^5$ and $R^5$ has the significance given above,

b) for the manufacture of the compounds of formula I in which $R^1$ signifies $C_{1-4}$-alkyl, $R^2$ signifies hydroxy, R signifies hydrogen and $R^4$ signifies 3-pyridyl or 2-pyrazinyl, reducing a ketone of the general formula

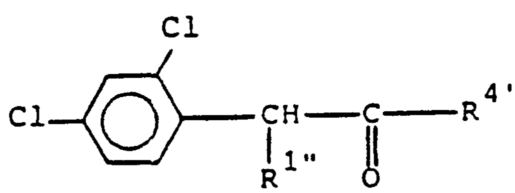

V

wherein $R^{4'}$ has the significance givenabove and $R^{1''}$ signifies $C_{1-4}$-alkyl,

c) for the manufacture of the compounds of formula I in which $R^1$ signifies $C_{1-4}$-alkyl, $R^2$ signifies hydroxy, R signifies $C_{1-4}$-alkyl and $R^4$ signifies 3-pyridyl or 2-pyrazinyl, reacting a ketone of general formula V, as defined above, with a compound of the general formula

$$R^{3'}-Y \qquad VI$$

wherein $R^{3'}$ signifies $C_{1-4}$-alkyl, Y signifies lithium or MgZ and Z signifies halogen,

d) for the manufacture of the compounds of formula I in which R signifies chlorine and $R^4$ signifies 3-pyridyl or 2-pyrazinyl, treating an alcohol of the general formula

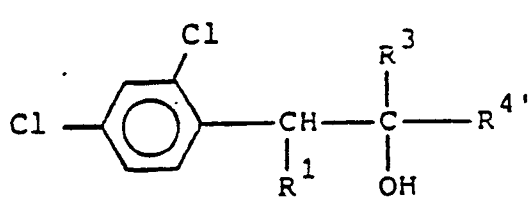

I'

wherein $R^1$, $R^3$ and $R^4$ have the significances give above,

or an epoxide of general formula II, as defined above, with a chlorinating agent,

e) for the manufacture of the compounds of formula I in which $R^1$ signifies $SR^5$ and $R^2$ signifies hydroxy, reacting a thioether of the general formula

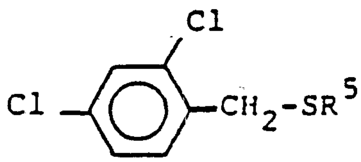

VII

wherein $R^5$ has the significance given above, with an aldehyde or ketone of the general formula

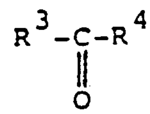

VIII

wherein $R^3$ and $R^4$ have the significances given above,

f) for the manufacture of the compounds of formula I in which $R^4$ signifies 3-pyridyl 1-oxide, 2-pyrazinyl 1-oxide, 2-pyrazinyl 4-oxide or 2-pyrazinyl 1,4-dioxide, N-oxidizing a pyridine or pyrazine derivative of the general formula

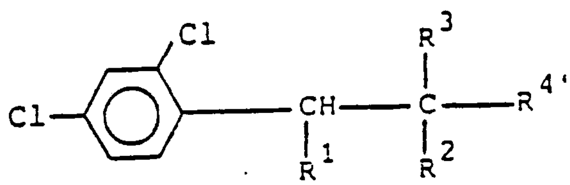

I"

wherein $R^1$, $R^2$, $R^3$ and $R^4$ have the significances given above,

and, if desired. reacting a compound of formula I obtained with an acid for the manufacture of the corresponding acid addition salt.

18. A process according to claim 17, characterized in that R in formula I signifies 3-pyridyl or 2-pyrazinyl and variant a), b), c) or d) is used.

19. A method for the control of fungi in agriculture and in horticulture, characterized by treating the locus to be protected with an effective amount of at least one of the compounds set forth in claims 1 to 9 or of one of the compositions set forth in claims 11 to 16.

20. The use of one of the compounds set forth in claims 1 to 9 or of one of the compositions set forth in claims 11 to 16 for the control of fungi in agriculture and in horticulture.

21. Compounds of the general formula

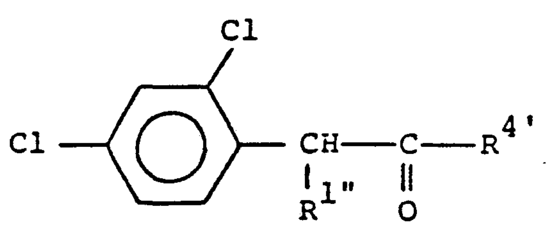

V

wherein $R^{1''}$ signifies $C_{1-4}$-alkyl

and $R^{4'}$ signifies 3-pyridyl or 2-pyrazinyl, as well as their N-oxides.

22. A fungicidal composition, characterized in that it contains an effective amount of at least one compound of the general formula

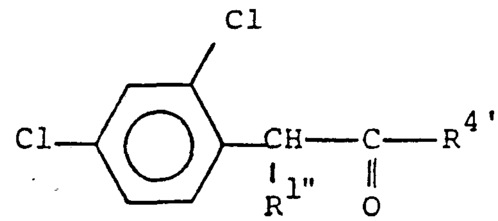

V

wherein $R^{1''}$ signifies $C_{1-4}$-alkyl and $R^{4'}$ signifies 3-pyridyl or 2-pyrazinyl or an N-oxide thereof as well as formulation adjuvants.

## Claims

for the Contracting State AT

1. A fungicidal composition, characterized in that it contains an effective amount of at least one compound of the general formula

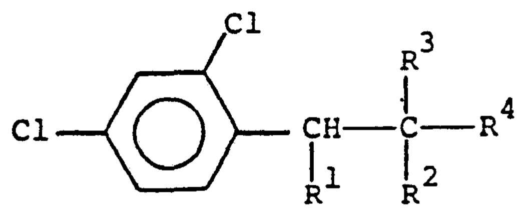

I

wherein $R^1$ signifies halogen, $C_{1-4}$-alkyl, $OR^5$, $SR^5$ or $OSO_2R^5$,
$R^2$ signifies hydroxy or chlorine,
$R^3$ signifies hydrogen or $C_{1-4}$-alkyl,
$R^4$ signifies 3-pyridyl, 3-pyridyl 1-oxide, 2-pyrazinyl, 2-pyrazinyl 1-oxide, 2-pyrazinyl 4-oxide or 2-pyrazinyl 1,4-dioxide
and $R^5$ signifies $C_{1-4}$-alkyl,
whereby $R^1$ can not be bromine or iodine where $R^2$ signifies chlorine and R signifies $C_{1\ 4}$-alkyl,
or an acid addition salt thereof as well as formulation adjuvants.

2. A fungicidal composition according to claim 1, wherein $R^4$ signifies 3-pyridyl or 2-pyrazinyl.

3. A fungicidal composition according to claim 1 or 2, wherein $R^3$ signifies hydrogen or methyl.

4. A fungicidal composition according to claim 2, characterized in that it contains an effective amount of α-(α,2,4-trichlorobenzyl)-α-methyl-3-pyridylmethanol as well as formulation adjuvants.

5. A fungicidal composition according to claim 2, characterized in that it contains an effective amount of α-(2,4-dichloro-α-methylbenzyl)-3-pyridylmethanol as well as formulation adjuvants.

6. A fungicidal composition according to claim 2, characterized in that it contains an effective amount of α-(α-bromo-2,4-dichlorobenzyl)-α-methyl-2-pyrazinylmethanol as well as formulation adjuvants.

7. A fungicidal composition according to claim 2, characterized in that it contains an effective amount of α-(α-bromo-2,4-dichlorobenzyl)-α-methyl-3-pyridylmethanol as well as formulation adjuvants.

8. A fungicidal composition according to claim 2, characterized in that it contains an effective amount of a compound selected from:
α-(2,4-Dichloro-α-methoxybenzyl)-α-methyl-3-pyridylmethanol,
2,4-dichloro-α-[1-hydroxy-1-(3-pyridyl)-ethyl]-benzyl methanesulphonate,
2,4-dichloro-α-[1-hydroxy-1-(2-pyrazinyl)-ethyl]-benzyl methanesulphonate,
α-(α,2,4-trichlorobenzyl)-α-ethyl-3-pyridylmethanol,
α-(α,2,4-trichlorobenzyl)-α-methyl-2-pyrazinylmethanol,
α-(α-iodo-2,4-dichlorobenzyl)-α-methyl-2-pyrazinylmethanol,
α-(2,4-dichloro-α-methylbenzyl)-α-methyl-3-pyridylmethanol,
α-(2,4-dichloro-α-methylbenzyl)-α-ethyl-3-pyridylmethanol,
α-(2,4-dichloro-α-ethylbenzyl)-α-methyl-3-pyridylmethanol,
α-(α,2,4-trichlorobenzyl)-α-methyl-3-pyridylmethyl chloride,
α-[2,4-dichloro-α-(methylthio)-benzyl]-3-pyridylmethanol,
α-(α,2,4-trichlorobenzyl)-α-methyl-3-pyridylmethyl chloride,
α-[2,4-dichloro-α-(methylthio)-benzyl]-α-methyl-3-pyridylmethanol and
α-[2,4-dichloro-α-(methylthio)-benzyl]-α-methyl-2-pyrazinylmethanol,
as well as formulation adjuvants.

9. A fungicidal composition according to claim 1, characterized in that it contains an effective amount of a compound selected from:
α-(2,4-Dichloro-α-ethylbenzyl)-3-pyridylmethanol,
3-(α,2,4-trichloro-ß-methylphenethyl)pyridine,
α-(2,4-dichloro-α-methylbenzyl)-3-pyridylmethanol 1-oxide and
α-(α,2,4-trichlorobenzyl)-α-methyl-3-pyridyl-methanol 1-oxide,
as well as formulation adjuvants.

10. A process for the manufacture of compounds of the general formula

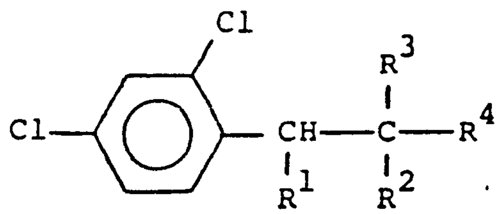

I

wherein $R^1$ signifies halogen, $C_{1-4}$-alkyl, $OR^5$, $SR^5$ or $OSO_2R^5$.
$R^2$ signifies hydroxy or chlorine,
$R^3$ signifies hydrogen or $C_{1-4}$-alkyl,
$R^4$ signifies 3-pyridyl, 3-pyridyl l-oxide, 2-pyrazinyl, 2-pyrazinyl 1-oxide, 2-pyrazinyl 4-oxide or 2-pyrazinyl 1,4-dioxide
and $R^5$ signifies $C_{1-4}$-alkyl,
whereby $R^1$ can not be bromine or iodine where $R^2$ signifies chlorine and $R^3$ signifies $C_{1-4}$-alkyl,
and of their acid addition salts, characterized by

a) for the manufacture of the compounds of formula I in which $R^1$ signifies halogen, $OR^5$, SR or $OSO2_{R,}$ $R^2$ signifies hydroxy and $R^4$ signifies 3-pyridyl or 2-pyrazinyl, reacting an epoxide of the general formula

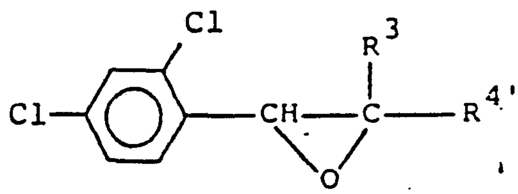

II

wherein $R^3$ has the significance given above
and $R^{4'}$ signifies 3-pyridyl or 2-pyrazinyl,
or an alcohol of the general formula

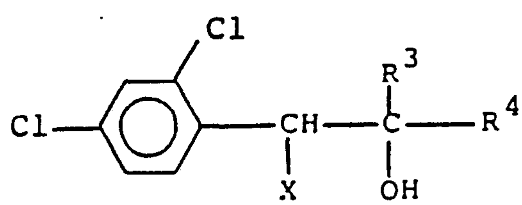

III

wherein $R^3$ and $R^{4'}$ have the significances given above
and X signifies an alkylsulphonate residue or arylsulphonate residue,
with a compound of the general formula

$$R^{1'}—H$$

IV

wherein $R^{1'}$ signifies halogen, $OR^5$, $SR^5$ or $OSO^2R^5$
and $R^5$ has the significance given above,

b) for the manufacture of the compounds of formula I in which $R^1$ signifies $C_{1-4}$-alkyl, R signifies hydroxy, $R^3$ signifies hydrogen and R signifies 3-pyridyl or 2-pyrazinyl, reducing a ketone of the general formula

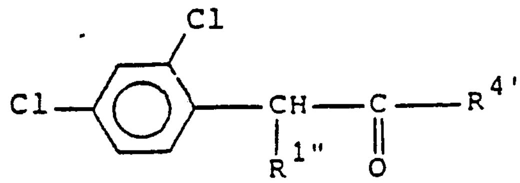

V

wherein $R^{4'}$ has the significance given above and $R^{1''}$ signifies $C_{1-4}$-alkyl,

c) for the manufacture of the compounds of formula I in which $R^1$ signifies $C_{1-4}$-alkyl, $R^2$ signiifies hydroxy, $R^3$ signifies $C_{1-4}$-alkyl and $R^4$ signifies 3-pyridyl or 2-pyrazinyl, reacting a ketone of general formula V as defined above, with a compound of the general formula

$$R^{3'}—Y \qquad VI$$

wherein $R^{3'}$ signifies $C_{1-4}$-alkyl,
Y signifies lithium or MgZ
and Z signifies halogen,

d) for the manufacture of the compounds of formula I in which $R^2$ signifies chlorine and $R^4$ signifies 3-pyridyl or 2-pyrazinyl, treating an alcohol of the general formula

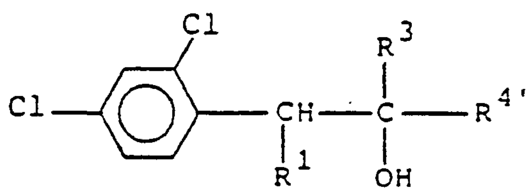

I'

wherein $R^1$, $R^3$ and $R^4$ have the significances give above,
or an epoxide of general formula II, as defined above, with a chlorinating agent,

e) for the manufacture of the compounds of formula I in which $R^1$ signifies $SR^5$ and $R^2$ signifies hydroxy, reacting a thioether of the general formula

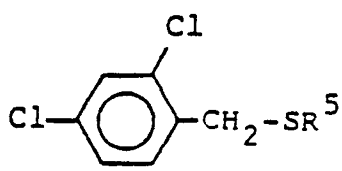

VII

wherein $R^5$ has the significance given above, with an aldehyde or ketone of the general formula

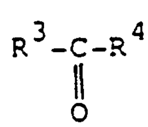

VIII

wherein $R^3$ and $R^4$ have the significances given above,

f) for the manufacture of the compounds of formula I in which R signifies 3-pyridyl 1-oxide, 2-pyrazinyl 1-oxide, 2-pyrazinyl 4-oxide or 2-pyrazinyl 1,4-dioxide, N-oxidizing a pyridine or pyrazine derivative of the general formula

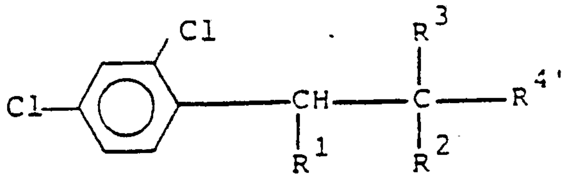

I"

wherein $R^1$, $R^2$, $R^3$ and $R^4$ have the significances given above,

and, if desired, reacting a compound of formula I obtained with an acid for the manufacture of the corresponding acid addition salt.

11. A process according to claim 10, characterized in that R in formula I signifies 3-pyridyl or 2-pyrazinyl and variant a), b), c) or d) is used.

12. A process for the manufacture of a fungicidal composition, characterized by mixing at least one of the compounds set forth in claims 1 to 9 with formulation adjuvants.

13. A method for the control of fungi in agriculture and in horticulture, characterized by treating the locus to be protected with an effective amount of one of the compounds or compositions set forth in claims 1 to 9.

14. The use of one of the compounds or compositions set forth in claims 1 to 9 for the control of fungi in agriculture and in horticulture.

15. A fungicidal composition, characterized in that it contains an effective amount of at least one compound of the general formula

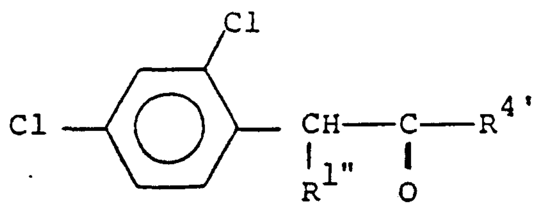

V

wherein $R^{1''}$ signifies $C_{1-4}$-alkyl

and $R^{4'}$ signifies 3-pyridyl or 2-pyrazinyl, or an N-oxide thereof as well as formulation adjuvants.

**Revendications**

pour les états contractants BE, CH, DE, FR, GB, IT, LI, NL.

1. - Composés de formule générale

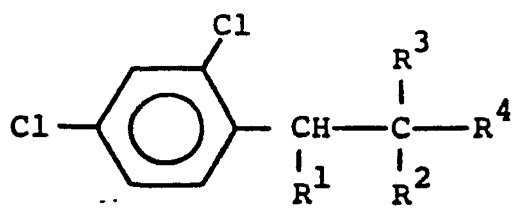

I

dans laquelle

$R^1$ représente un halogène, un groupe alkyle en C1-C4, $OR^5$, $SR^5$ ou $OSO_2R^5$,

$R^2$ représente un groupe hydroxy ou le chlore,

$R^3$ représente l'hydrogène ou un groupe alkyle en C1-C4,

$R^4$ représente un reste 3-pyridyle, 1-oxyde de 3-pyridyle, 2-pyrazinyle, 1-oxyde de 2-pyrazinyle, 4-oxyde de 2-pyrazinyle ou 1,4-dioxyde de 2-pyrazinyle et

$R^5$ représente un groupe alkyle en C1-C4, étant spécifié que, lorsque $R^2$ représente le chlore et $R^3$ un groupe alkyle en C1-C4, $R^1$ ne peut représenter le brome ou l'iode,

et leurs sels formés par addition avec des acides.

2. - Composés selon la revendication 1, dans lesquels $R^4$ représente un reste 3-pyridyle ou 2-pyrazinyle.

3. - Composés selon la revendication 1 ou 2, dans lesquels $R^3$ représente l'hydrogène ou un groupe méthyle.

4. - L'alpha-(alpha,2,4-trichlorobenzyl)-alpha-méthyl-3-pyridyl-méthanol.

5. - L'alpha-(2,4-dichloro-alpha-méthylbenzyl)-3-pyridyl-méthanol.

6. - L'alpha-(alpha-bromo-2,4-dichlorobenzyl)-alpha-méthyl-2-pyrazinyl-méthanol.

7. - L'alpha-(alpha-bromo-2,4-dichlorobenzyl)-alpha-méthyl-3-pyridyl-méthanol.

8. - Un composé selon la revendication 2, choisi parmi les suivants:

l'alpha-(2,4-dichloro-alpha-méthoxybenzyl)-alpha-méthyl-3-pyridyl-méthanol,

le méthane-sulfonate de 2,4-dichloro-alpha-|1-hydroxy-1-(3-pyridyl)-éthyl|-benzyle,

le méthane-sulfonate de 2,4-dichloro-alpha-|1-hydroxy-1-(2-pyrazinyl)-éthyl|-benzyle,

l'alpha-(alpha,2,4-trichlorobenzyl)-alpha-éthyl-3-pyridyl-méthanol,

l'alpha-(alpha,2,4-trichlorobenzyl)-alpha-méthyl-2-pyrazinyl-méthanol,

l'alpha-(alpha-iodo-2,4-dichlorobenzyl)-alpha-méthyl-2-pyrazinyl-méthanol,

l'alpha-(2,4-dichloro-alpha-méthylbenzyl)-alpha-méthyl-3-pyridyl-méthanol,

l'alpha-(2,4-dichloro-alpha-méthylbenzyl)-alpha-éthyl-3-pyridyl-méthanol,

l'alpha,(2,4-dichloro-alpha-éthylbenzyl)-alpha-méthyl-3-pyridyl-méthanol,
le chlorure d'alpha-(alpha,2,4-trichloro-benzyl)-alpha-méthyl-3-pyridyl-méthyle,
l'alpha-|2,4-dichloro-alpha-(méthylthio)-benzyl|-3-pyridyl-méthanol,
le chlorure d'alpha-(alpha,2,4-trichloro-benzyl)-alpha-méthyl-3-pyridyl-méthyle,
l'alpha-|2,4-dichloro-alpha-(méthylthio)-benzyl|-alpha-méthyl-3-pyridyl-méthanol et
l'alpha-|2,4-dichloro-alpha-(méthylthio)-benzyl|-alpha-méthyl-2-pyrazinyl-méthanol.

9. - Un composé selon la revendication 1, choisi parmi les suivants:
l'alpha-(2,4-dichloro-alpha-éthylbenzyl)-3-pyridyl-méthanol,
la 3-(alpha,2,4-trichloro-bêta-méthylphénéthyl)-pyridine,
le 1-oxyde de l'alpha-(2,4-dichloro-alpha-méthylbenzyl)-3-pyridyl-méthanol et
le 1-oxyde de l'alpha-(alpha,2,4-trichloro-benzyl)-alpha-méthyl-3-pyridyl-méthanol.

10. - Composés selon l'une des revendications 1 à 9, en tant que substances actives fongicides.

11. - Produit fongicide caractérisé en ce qu'il contient une quantité efficace d'au moins un composé de formule générale

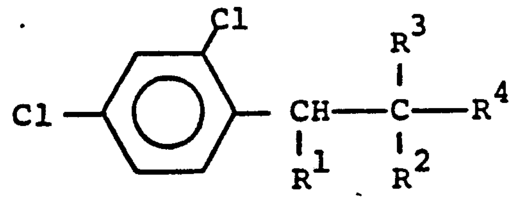

I

dans laquelle:
$R^1$ représente un halogène, un groupe alkyle en C1-C4, $OR^5$, $SR^5$ ou $OSO_2R^5$,
$R^2$ représente un groupe hydroxy ou le chlore,
$R^3$ représente l'hydrogène ou un groupe alkyle en C1-C4,
$R^4$ représente un reste 3-pyridyle, 1-oxyde de 3-pyridyle, 2-pyrazinyle, 1-oxyde de 2-pyrazinyle, 4-oxyde de 2-pyrazinyle ou 1,4-dioxyde de 2-pyrazinyle et
$R^5$ représente un groupe alkyle en Cl-C4,
étant spécifié que, lorsque $R^2$ représente le chlore et $R^3$ un groupe alkyle en C1-C4, $R^1$ ne peut représenter le brome ou l'iode,
ou un sel d'un tel composé formé par addition avec un acide, avec des produits auxiliaires de formulation.

12. - Produit fongicide selon la revendication 11, dans lequel $R^4$ représente un reste 3-pyridyle ou 2-pyrazinyle.

13. - Produit fongicide selon la revendication 12, caractérisé en ce qu'il contient une quantité efficace d'alpha-(alpha,2,4-trichlorobenzyl)-alpha-méthyl-3-pyridyl-méthanol et des produits auxiliaires de formulation.

14. - Produit fongicide selon la revendication 12, caractérisé en ce qu'il contient une quantité efficace d'alpha-(2,4-dichloro-alpha-méthylbenzyl)-3-pyridyl-méthanol et des produits auxiliaires de formulation.

15. - Produit fongicide selon la revendication 12, caractérisé en ce qu'il contient une quantité efficace d'alpha-(alpha-bromo-2,4-dichlorobenzyl)-alpha-méthyl-2-pyrazinyl-méthanol et des produits auxiliaires de formulation.

16. - Produit fongicide selon la revendication 12, caractérisé en ce qu'il contient une quantité efficace d'alpha-(alpha-bromo-2,4-dichlorobenzyl)-alpha-méthyl-3-pyridyl-méthanol et des produits auxiliaires de formulation.

17. - Procédé de préparation des composés de formule générale

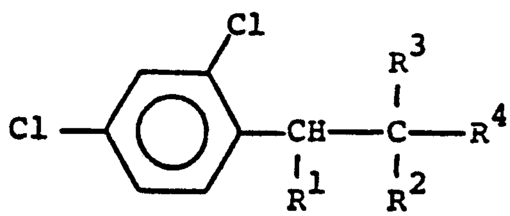

I

dans laquelle:
$R^1$ représente un halogène, un groupe alkyle en C1-C4, $OR^5$, $SR^5$ ou $OSO_2R^5$,
$R^2$ représente un groupe hydroxy ou le chlore,
$R^3$ représente l'hydrogéne ou un groupe alkyle en Cl-C4,
$R^4$ représente un reste 3-pyridyle, 1-oxyde de 3-pyridyle, 2-pyrazinyle, 1-oxyde de 2-pyrazinyle, 4-oxyde de 2-pyrazinyle ou 1,4-dioxyde ds 2-pyrazinyle et
$R^5$ représente un groupe alkyle en Cl-C4, étant spécifié que, lorsque $R^2$ représente le chlors et $R^3$ un groupe alkyle en Cl-C4, $R^1$ ne peut représenter le brome ou l'iode,
et de leurs sels formés par addition avec des acides, caractérisé en ce que

a) pour préparer les composés de formule I dans laquelle $R^1$ représente un halogène, un groupe $OR^5$, $SR^5$ ou $OSO_2R^5$, $R^2$ représente un groupe hydroxy st $R^4$ un rests 3-pyridyls ou 2-pyrazinyls, on fait réagir un époxyds de formule générale

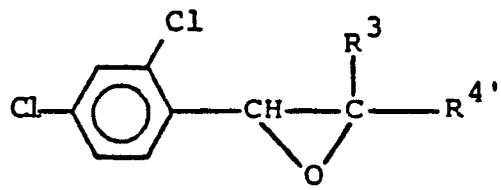

dans laquelle $R^3$ a les significations indiquées cidessus et $R^{4'}$représents un reste 3-pyridyls ou 2-pyrazinyls, ou un alcool de formuls générale

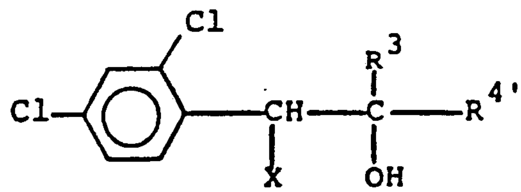

III

dans laquelle:
$R^3$ et $R^4$ ont les significations indiquées ci-dessus et
X représente un rests alkylsulfonate ou arylsulfonate,
avec un composé de formule générale

$R^{1'}$———H IV

dans laquelle:
$R^1$ représsnts un halogèns, un groupe $OR^5$, $SR^5$ ou $OSO_2R^5$ et
$R^5$ a les significations indiquéss ci-dessus,
b) pour préparer les composés de formule
I dans laquelle $R^1$ représente un groupe alkyle en C1-C4, $R^2$ un groups hydroxy, $R^3$ l'hydrogène et $R^4$ un reste 3-pyridyle ou 2-pyrazinyls, on réduit une cétons de formule générale

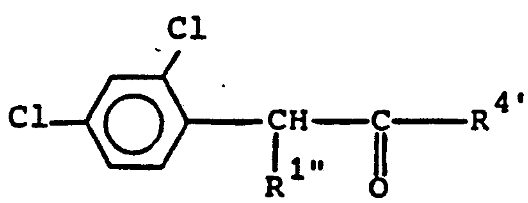

V

dans laquelle:
$R^{4'}$ a les significations indiquées ci-dessus
et $R^{1''}$ représente un groups alkyls en C1-C4,
c) pour préparer les composés de formule I dans laquelle $R^1$ représente un groupe alkyle en Cl-C4, $R^2$ un groupe hydroxy, $R^3$ un groupe alkyle en Cl-C4 et $R^4$ un reste 3-pyridyle ou 2-pyrazinyle, on fait réagir une cétone de formule générale V définie cidessus, avec un composé de formule générale

$R^{3'}$———Y VI

dans laquelle:
$R_{3'}$ représente un groupe alkyle en C1-C4,
Y représente le lithium ou MgZ
et Z représente un halogène,

d) pour préparer les composés de formule I dans laquelle $R^2$ représente le chlore et $R^4$ un reste 3-pyridyle ou 2-pyrazinyle, on traite un alcool de formule générale

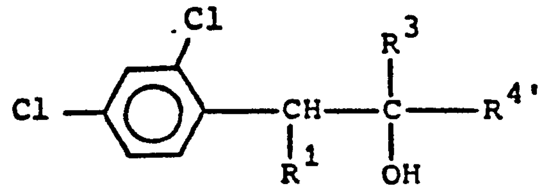

I'

dans laquelle:

$R^1$, $R^3$ et $R^4$ ont les significations indiquées ci-dessus,

ou un époxyde de formule générale II définie ci-dessus, par un agent chlorant,

e) pour préparer les composés de formule I dans laquelle $R^1$ représente un groupe $SR^5$ et $R^2$ un groupe hydroxy, on fait réagir un thioéther de formule générale

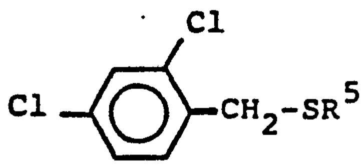

VII

dans laquelle:

$R^5$ a les significations indiquées ci-dessus, avec un aldéhyde ou une cétone respectivement, de formule générale

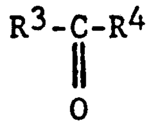

VIII

dans laquelle:

$R^3$ et $R^4$ ont les significations indiquées ci-dessus,

f) pour préparer les composés de formule I dans laquelle $R^4$ représente un reste 1-oxyde de 3-pyridyle, 1-oxyde de 2-pyrazinyle, 4-oxyde de 2-pyra-zinyle ou 1,4-dioxyde de 2-pyrazinyle, on soumet respectivement un dérivé de pyridine ou de pyrazine de formule générale

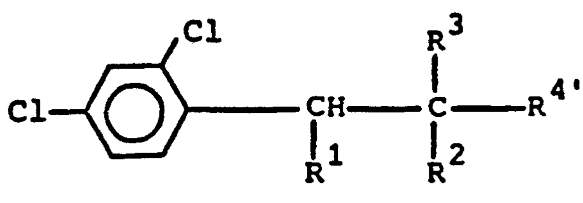

I"

dans laquelle:

$R^1$, $R^2$, $R^3$ et $R^4$ ont les significations indiquées ci-dessus,

à N-oxydation et

si on le désire, on fait réagir un composé obtenu, répondant à la formule 1, avec un acide, pour préparation du sel correspondant formé par addition avec un acide.

18. - Procédé selon la revendication 17, caractérisé en ce que le symbole $R^4$ de la formule 1 représente un reste 3-pyridyle ou 2-pyrazinyle et en ce que l'on applique la variante a), b), c) ou d).

19. - Procédé pour combattre les mycètes dans l'agriculture et l'horticulture, caractérisé en ce que l'on traite le bien à protéger par une quantité efficace d'au moins un des composés mentionnés dans les revendications 1 à 9 ou d'un produit mentionné dans les revendications 11 à 16.

20. - Utilisation de l'un des composés mentionnés dans les revendications 1 à 9 ou de l'un des produits mentionnés dans les revendications 11 à 16 dans la lutte contre les mycètes en agriculture et en horticulture.

21. - Composés de formule générale

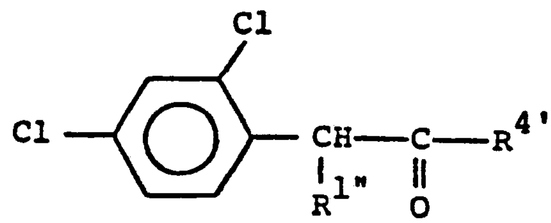

V

dans laquelle:
$R^{1''}$ représente un groupe alkyle en C1-C4
et $R^{4'}$ représente un reste 3-pyridyle ou 2-pyrazinyle,
et leurs N-oxydes.

22. - Produit fongicide caractérisé en ce qu'il contient une quantité efficace d'au moins un composé de formule générale

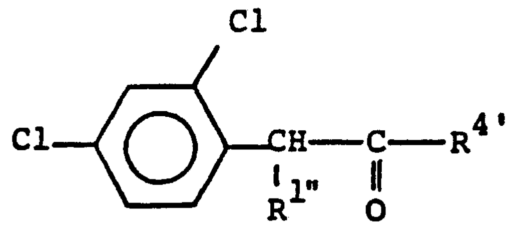

V

dans laquelle:
$R^{1''}$ représente un groupe alkyle en C1-C4
et $R^{4'}$ représente un groupe 3-pyridyle ou 2-pyrazinyle,
ou d'un N-oxyde d'un tel composé, avec des produits auxiliaires de formulation.

**Revendications**

pour l'etat contractant AT

1. - Produit fongicide caractérisé en ce qu'il contient une quantité efficace d'au moins un composé de formule générale

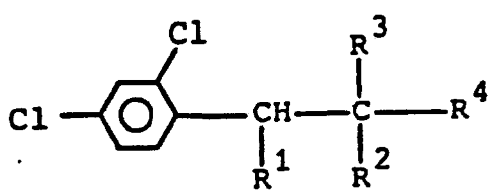

I

dans laquelle:
$R^1$ représente un halogène un groupe alkyle en C1-C4, $OR^5$, $SR^5$ ou $OSO_2R^5$
$R^2$ représente un groupe hydroxy ou le chlore,
$R^3$ représente l'hydrogène ou un groupe alkyle en C1-C4,
$R^4$ représente un reste 3-pyridyle, 1-oxyde de 3-pyridyle, 2-pyrazinyle, 1-oxyde de 2-pyrazinyle, 4-oxyde de 2-pyrazinyle ou 1,4-dioxyde de 2-pyrazinyle et
$R^5$ représente un groupe alkyle en Cl-C4,
étant spécifié que, lorsque $R^2$ représente le chlore et $R^3$ un groupe alkyle en Cl-C4, $R^1$ ne peut représenter le brome ou l'iode,
ou d'un sel d'un tel composé formé par addition avec un acide, avec des produits auxiliaires de formulation.

2. - Produit fongicide selon la revendication 1, dans lequel $R^4$ représente un reste 3-pyridyle ou 2-pyrazinyle

3. - Produit fongicide selon la revendication 1 ou 2, dans lequel $R^3$ représente l'hydrogène ou un groupe méthyle.

4. - Produit fongicide selon la revendication 2, caractérisé en ce qu'il contient une quantité efficace d'alpha-(alpha,2,4-trichlorobenzyl)-alpha-méthyl-3-pyridyl-méthanol et des produits auxiliaires de formulation.

5. - Produit fongicide selon la revendication 2, caractérisé en ce qu'il contient une quantité efficace d'alpha-(2,4-dichloro-alpha-méthylbenzyl)-3-pyridyl-méthanol et des produits auxiliaires de formulation.

6. - Produit fongicide selon la revendication 2, caractérisé en ce qu'il contient une quantité efficace d'alpha-(alpha-bromo-2,4-dichlorobenzyl)-alpha-méthyl-2-pyrazinyl-méthanol et des produits auxiliaires de formulation.

7. - Produit fongicide selon la revendication 2, caractérisé en ce qu'il contient une quantité efficace d'alpha-(alpha-bromo-2,4-dichlorobenzyl)-alpha-méthyl-3-pyridyl-méthanol et des produits auxiliaires de formulation.

8. - Produit fongicide selon la revendication 2, caractérisé en ce qu'il contient une quantité efficace d'un composé choisi parmi les suivants:
l'alpha-(2,4-dichloro-alpha-méthoxybenzyl)-alpha-méthyl-3-pyridyl-méthanol,
le méthane-sulfonate de 2,4-dichloro-alpha-|1-hydroxy-1-(3-pyridyl)-éthyl|-benzyle,
le méthane-sulfonate de 2,4-dichloro-alpha-|1-hydroxy-1-(2-pyrazinyl)-éthyl|-benzyle,
l'alpha-(alpha,2,4-trichlorobenzyl)-alpha-éthyl-3-pyridyl-méthanol,
l'alpha-(alpha,2,4-trichlorobenzyl)-alpha-méthyl-2-pyrazinyl-méthanol,
l'alpha-(alpha-iodo-2,4-dichlorobenzyl)-alpha-méthyl-2-pyrazinyl-méthanol,
l'alpha-(2,4-dichloro-alpha-méthylbenzyl)-alpha-méthyl-3-pyridyl-méthanol,
l'alpha-(2,4-dichloro-alpha-méthylbenzyl)-alpha-éthyl-3-pyridyl-méthanol,
l'alpha-(2,4-dichloro-alpha-éthylbenzyl)-alpha-méthyl-3-pyridyl-méthanol,
le chlorure d'alpha-(alpha,2,4-trichloro-benzyl)-alpha-méthyl-3-pyridyl-méthyle,
l'alpha-|2,4-dichloro-alpha-(méthylthio)-benzyl|-3-pyridyl-méthanol,
le chlorure d'alpha-(a1pha,2,4-trichloro-benzyl)-alpha-méthyl-3-pyridyl-méthyle,
l'alpha-|2,4-dichloro-alpha-(méthylthio)-benzyl|-alpha-méthyl-3-pyridyl-méthanol et
l'alpha-|2,4-dichloro-alpha-(méthylthio)
benzyl|-alpha-méthyl-2-pyrazinyl-mèthanol-et des produits auxiliaires de formulation.

9. - Produit fongicide selon la revendication 1, caractérisé en ce qu'il contient une quantité efficace d'un composé choisi parmi les suivants:
l'alpha-(2,4-dichloro-alpha-éthylbenzyl)-3-pyridyl-méthanol,
la 3-(alpha,2,4-trichloro-bêta-méthylphénéthyl)-pyridine,
le 1-oxyde de l'alpha-(2,4-dichloro-alpha-méthylbenzyl)-3-pyridyl-méthanol et
le 1-oxyde de l'alpha-(a1pha,2,4-trichloro-benzyl)-alpha-méthyl-3-pyridyl-méthanol. et des produits auxiliaires de formulation.

10. - Procédé de préparation des composés de formule générale

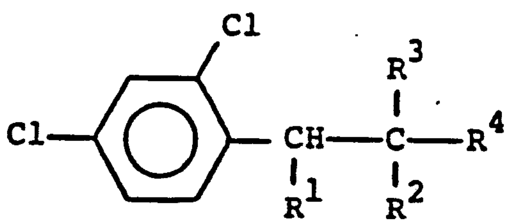

I

dans laquelle:
$R^1$ représente un halogène, un groupe alkyle en C1-C4, $OR^5$, $SR^5$ ou $OSO_2R^5$,
$R^2$ représente un groupe hydroxy ou le chlore,
$R^3$ représente l'hydrogène ou un groupe alkyle en C1-C4,
$R^4$ représente un reste 3-pyridyle, 1-oxyde dé 3-pyridyle, 2-pyrazinyle, 1-oxyde de 2-pyrazinyle, 4-oxyde de 2-pyrazinyle ou 1,4-dioxyde de 2-pyrazinyle et
$R^5$ représente un groupe alkyle en CL-C4,
étant spécifié que, lorsque $R^2$ représente le chlore et $R^3$ un groupe alkyle en C1-C4, $R^1$ ne peut représenter le brome ou l'iode,
et de leurs sels formés par addition avec des acides, caractérisé en ce que:
a) pour préparer les composés de formule 1 dans laquelle $R^1$ représente un halogène, un groupe $OR^5$, $SR^5$ ou $OSO_2R^5$, $R^2$ représente un groupe hydroxy et $R^4$ un reste 3-pyridyle ou 2-pyrazinyle, on fait réagir un époxyde de formule générale

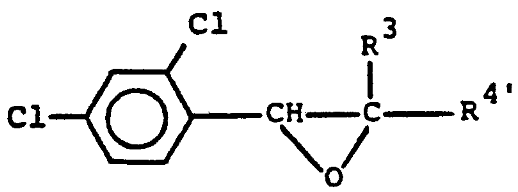

II

dans laquelle $R^3$ a les significations indiquées cidessus et $R^{4'}$représente un reste 3-pyridyle ou 2-pyrazinyle, ou un alcool de formule générale

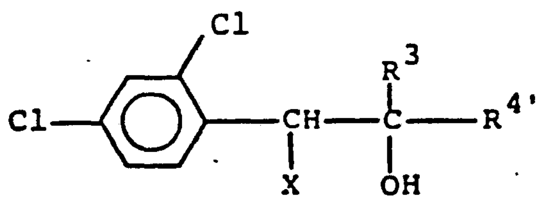

III

dans laquelle:
$R^3$ et $R^4$ ont les significations indiquées ci-dessus et
X représente un reste alkylsulfonate ou arylsulfonate,
avec un composé de formule générale

$$R^{1'}\text{———}H \qquad IV$$

dans laquelle:
$R^1$ représente un halogène, un groupe $OR^5$, $SR^5$ ou $OSO_2R^5$ et
$R^5$ a les significations indiquées ci-dessus,

b) pour préparer les composés de formule I dans laquelle $R^1$ représente un groupe alkyle en Cl-C4, $R^2$ un groupe hydroxy, $R^3$ l'hydrogène et $R^4$ un reste 3-pyridyle ou 2-pyrazinyle, on réduit une cétone de formule générale

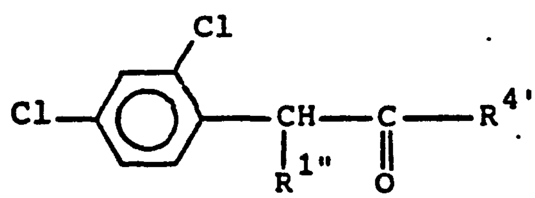

V

dans laquelle:
$R^{4'}$ a les significations indiquées ci-dessus
et $R^{1''}$ représente un groupe alkyle en C1-C4,

c) pour préparer les composés de formule I dans laquelle $R^1$ représente un groupe alkyle en C1-C4, $R^2$ un groupe hydroxy, $R^3$ un groupe alkyle en C1-C4 et $R^4$ un reste 3-pyridyle ou 2-pyrazinyle, on fait réagir une cétone de formule générale V définie cidessus, avec un composé de formule générale

$$R^{3'}\text{———}Y \qquad VI$$

dans laquelle:
$R^3$ représente un groupe alkyle en Cl-C4,
Y représente le lithium ou MgZ
et Z représente un halogène,

d) pour préparer les composés de formule I dans laquelle $R^2$ représente le chlore et $R^4$ un reste 3-pyridyle ou 2-pyrazinyle, on traite un alcool de formule générale

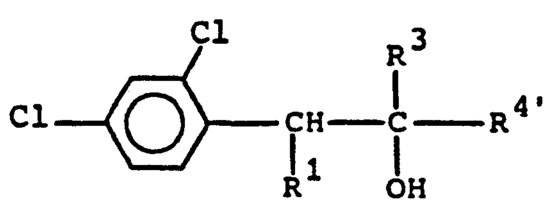

I'

dans laquelle:
$R^1$, $R^3$ et $R^4$ ont les significations indiquées ci-dessus,
ou un époxyde de formule générale II définie ci-dessus, par un agent chlorant,

e) pour préparer les composés de formule I dans laquelle $R^1$ représente un groupe $SR^5$ et $R^2$ un groupe hydroxy, on fait réagir un thioéther de formule générale

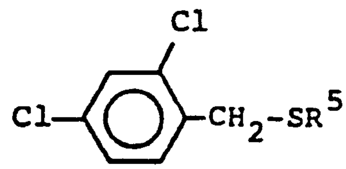

VII

dans laquelle:
$R^5$ a les significations indiquées ci-dessus, avec un aldéhyde ou une cétone respectivement, de formule générale

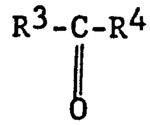

VIII

dans laquelle:
$R^3$ et $R^4$ ont les significations indiquées ci-dessus,
f) pour préparer les composés de formule I dans laquelle $R^4$ représente un reste 1-oxyde de 3-pyridyle, 1-oxyde de 2-pyrazinyle, 4-oxyde de 2-pyra-zinyle ou 1,4-dioxyde de 2-pyrazinyle, on soumet respectivement un dérivé de pyridine ou de pyrazine de formule générale

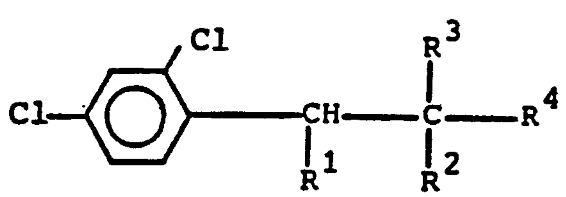

I"

dans laquelle:
$R^1$, $R^2$, $R^3$ et $R^4$ ont les significations indiquées ci-dessus,
à N-oxydation et
si on le désire, on fait réagir un composé obtenu, répondant à la formule I, avec un acide, pour préparation du sel correspondant formé par addition avec un acide.

11. - Procédé selon la revendication 10, caractérisé en ce que le symbole $R^4$ de la formule représente un reste 3-pyridyle ou 2-pyrazinyle et en ce que l'on applique la variante a), b), c) ou d).

12. - Procédé de préparation d'un produit fongicide, caractérisé en ce que l'on mélange au moins un des composés mentionnés dans les revendications 1 à 9 avec des produits auxiliaires de formulation.

13. - Procédé pour combattre les mycètes dans l'agriculture et l'horticulture, caractérisé en ce que l'on traite le bien à protéger par une quantité efficace d'au moins un des composés ou produits mentionnés dans les revendications 1 à 9.

14. - Utilisation d'un des composés ou produits mentionnés dans les revendications 1 à 9 pour la lutte contre les mycètes dans l'agriculture et l'horticulture.

15. - Produit fongicide caractérisé en ce qu'il contient une quantité efficace d'au moins un composé de formule générale

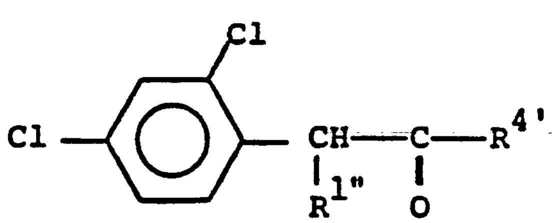

V

dans laquelle:
$R^1$ représente un groupe alkyle en Cl-C4
et $R^4$ représente un reste 3-pyridyle ou 2-pyrazinyle ou d'un N-oxyde d'un tel composé, avec des produits auxiliaires de formulation.